# EUROPEAN PATENT APPLICATION

(11) **EP 4 844 064 A1**
(43) Date of publication of application: **25.11.2026**
(21) Application number: 25741632.1
(22) Date of filing: 17.01.2025
(51) Int. Cl.: C12N 15/113, C12N 15/11, A61P 11/00, A61P 35/00, A61P 9/00, A61P 13/12

(54) **RNAI AGENT FOR INHIBITING LYSOPHOSPHATIDIC ACID RECEPTOR 1 (LPAR1) GENE EXPRESSION, AND USE THEREOF**

(30) Priority: 19.01.2024 CN 202410083239
(71) Applicant: YUN HO BIO CO., LTD, Shanghai 201206 (CN)
(72) Inventor: WU, Yun, Shanghai 201206 (CN); ZHU, Yun, Shanghai 201206 (CN); MAO, Song, Shanghai 201206 (CN); LIU, Yang, Shanghai 201206 (CN); DONG, Xianglei, Shanghai 201206 (CN)
(74) Representative: Simmons & Simmons LLP (Munich)
(86) International application number: PCT/CN2025/073044
(87) International publication number: WO 2025/153070

(57) **Abstract**

Disclosed in the present invention are an RNAi agent for inhibiting LPAR1 gene expression in cells, and a pharmaceutical composition thereof. The present invention further relates to the therapeutic use of the RNAi agent.

## Description

### Technical Filed

The present invention relates to nucleic acids that inhibit the expression of lysophosphatidic acid receptor 1 (LPAR1) gene in cells, including RNAi agents (such as siRNA), and pharmaceutical compositions comprising the same. The invention also relates to therapeutic uses of the RNAi agents.

### Background

Lysophosphatidic acid (LPA) is a bioactive phospholipid generated during cell membrane synthesis, present in all eukaryotic tissues and biological fluids (including blood, cerebrospinal fluid, semen, urine, saliva, aqueous humor, etc.), and detectable under both physiological and pathological conditions. LPA can be detected under neuropathic injury conditions, in serum of patients with systemic sclerosis (SSc), in sepsis, in ascites associated with pancreatic cancer, and in plasma of patients suffering from chronic liver injury and obesity-related diseases. LPA mediates its effects through six specific G protein-coupled receptors (LPAR1 to LPAR6) and is implicated in a variety of cellular responses (such as proliferation and migration) as well as in the pathological processes of multiple diseases, including fibrosis, cancer, neurological disorders, and bone metabolism.

LPA biosignaling is mediated through intracellular receptors (nuclear receptors, peroxisome proliferator-activated receptor gamma (PPAR), actin-binding proteins (ABP)) and extracellular receptors (LPAR1-6). Among these, the LPA receptor subtypes (LPAR1-6) are classified into the endothelial differentiation gene (Edg) family of G protein-coupled receptors (GPCRs) and the non-Edg family of purinergic receptors. The first three subtypes of LPA receptors (LPAR1-3) belong to the Edg family (Edg2, Edg4, Edg7) of GPCRs, and share similar amino acid sequences. In contrast, LPAR4 (GPR23/P2Y9), LPAR5 (GPR92), and LPAR6 (GPR87) belong to the non-Edg subfamily cluster of purinergic GPCRs.

LPAR1 was first identified in 1996 in the developing brain, primarily enriched in the ventricular zone of the embryonic cerebral cortex, and was also found in the marginal zone and meninges, and was subsequently established as a receptor for LPA. LPAR1 is widely expressed in various tissues and organs of the human body, with relatively higher mRNA levels in the brain, heart, colon, small intestine, and placenta, and lower levels in other organs and tissues. LPA induces the complex formed by LPAR1 (also known as Edg2) receptor coupling with Gα i/o, Gα q/11, and Gα12/13, which transduces signals through molecules such as Ras homolog (Rho) family GTPases, phospholipase C (PLC), diacylglycerol (DAG), mitogen-activated protein kinase (MAPK), and phosphatidylinositol 3-kinase (PI3K)-protein kinase B (Akt). LPA mediates a variety of functions through G protein-coupled LPAR1, including cell survival, cell proliferation, cell adhesion, cell migration, cytoskeletal changes, Ca²⁺ mobilization, immune function, and myelination. For example, it promotes astrocyte proliferation and neuronal differentiation, proliferation of oligodendrocytes and smooth muscle cells, migration and anti-apoptosis of Schwann cells, and mineralization and osteogenic transformation of valvular interstitial cells (VICs). Previous studies have demonstrated that aberrant LPA-LPAR1 signaling can lead to a spectrum of diseases, including neuropathic pain, neurodevelopmental disorders, neuropsychiatric and neurodegenerative diseases, cardiovascular diseases, skeletal disorders, fibrosis, cancer, infertility, and obesity. Pulmonary fibrosis is a common pathological process that can be induced by more than 100 clinical conditions. For instance, idiopathic pulmonary fibrosis (IPF), the most prevalent and severe interstitial lung disease, may be triggered by allergens, toxic chemicals, radiation, other persistent irritants, and unknown factors. Numerous studies have demonstrated that abnormally elevated LPA expression levels in bronchoalveolar lavage fluid are associated with IPF. It has been shown that bone marrow-derived cells represent an important source of myofibroblasts in fibrotic organs. In a bleomycin-induced lung injury model, migrated bone marrow-derived mesenchymal stem cells (BMSCs) significantly contribute to the formation of α-smooth muscle actin (α-SMA)-positive myofibroblasts. Importantly, LPAR1 antagonists can attenuate pulmonary fibrosis by inhibiting the differentiation of BMSCs into myofibroblasts and the secretion of extracellular matrix (ECM).

Hepatic fibrosis (HF) is a pathological process in the progression of chronic liver diseases, characterized by the recurrent destruction and subsequent regeneration of hepatocytes, along with the diffuse excessive deposition and abnormal distribution of extracellular matrix (ECM) components, including collagen, glycoproteins, and proteoglycans, within the liver. Severe fibrosis can lead to disorganization of tissue architecture and liver failure. The primary source of ECM is hepatic stellate cells (HSCs), which become activated into myofibroblasts upon liver injury. Activated myofibroblasts, mainly characterized by the expression of α-smooth muscle actin (α-SMA), play a critical role in fibrogenesis. Studies have found that LPA stimulates the proliferation of both hepatocytes and HSCs. In chronic hepatitis C-induced liver fibrosis, plasma concentrations of LPA are elevated. Research has shown that silymarin, caffeine, and their combination reduce the degree of hepatic fibrosis by significantly decreasing the expression of hepatic LPAR1, TGF-β1, CTGF, and α-SMA genes, thereby improving liver function. Furthermore, it has been demonstrated that blockade of LPAR1 inhibits liver fibrosis in rodent models of nonalcoholic steatohepatitis (NASH).

Renal fibrosis is generally caused by an aberrant wound healing process. The wound healing response involves multiple cell types, ultimately leading to fibroblast expansion and activation into myofibroblasts. Studies have demonstrated that in the unilateral ureteral obstruction (UUO)-induced renal fibrosis model, LPAR1-specific LPA signaling induces the expression of connective tissue growth factor (CTGF) through the myocardin-related transcription factor (MRTF)-serum response factor (SRF) pathway, thereby mediating profibrotic epithelial-fibroblast communication, driving fibroblast proliferation and myofibroblast differentiation, and consequently causing renal fibrosis. Genetic ablation of *LPAR1* protects mice from UUO-induced renal fibrosis, and disruption of the LPA-LPAR1 pathway significantly reduces the accumulation of fibroblasts and myofibroblasts, thereby ameliorating renal fibrosis.

Hypertrophic cardiomyopathy (HCM) is the most common inherited cardiomyopathy. Patients with HCM are at risk for arrhythmias, heart failure, and stroke, and myocardial fibrosis is a major pathological feature of HCM. Studies have shown that in a mouse model of HCM, the *Lpar1* gene is predominantly expressed in lymphatic endothelial cells (LECs) and cardiac fibroblasts, and knockout of the Lpar1 gene reduces hypertrophy and fibrosis.

Currently, no small nucleic acid-based drugs targeting this gene have been approved for marketing, and there remains a need to develop such drugs with improved efficacy, prolonged durability, specific targeting, and/or safety.

### Summary

The present invention is directed to targeting the LPAR1 gene with RNAi agents, achieving efficient inhibition of target genes and proteins specifically in target organs, thereby blocking various diseases caused by aberrant LPA-LPAR1 signaling, such as fibrotic diseases (e.g., pulmonary fibrosis, hepatic fibrosis, renal fibrosis), hypertrophic cardiomyopathy, diffuse scleroderma, adult-onset scleroderma, cancers (e.g., hepatocellular carcinoma, ovarian cancer, glioblastoma), diabetic nephropathy, atherosclerosis, rheumatoid arthritis, neuropathic pain, non-alcoholic steatohepatitis, benign prostatic hyperplasia, and demyelination.

One aspect of the present invention provides an RNAi agent for inhibiting expression of the lysophosphatidic acid receptor 1 (LPAR1) gene in a cell, comprising a sense strand and an antisense strand forming a duplex region, wherein the antisense strand comprises at least 15, at least 16, at least 17, at least 18, at least 19, at least 20, or at least 21 contiguous nucleotides that are at least 80%, 85%, 90%, 95%, or 100% complementary to an equal-length contiguous nucleotide sequence of a target region of mRNA encoding LPAR1, or a nucleotide sequence having no more than 3 nucleotide differences therefrom.

In some embodiments, the target region is positions 602-631, 793-825, 833-859, 1107-1143, 1167-1194, 1211-1288, 1326-1360, 1489-1523, 1777-1804, 2264-2290, or 12786-2813 of the nucleotide sequence set forth in SEQ ID NO: 1.

In some embodiments, the target region is positions 602-628, 604-630, 605-631, 793-819, 794-820, 795-821, 796-822, 797-823, 799-825, 833-859, 1107-1133, 1108-1134, 1116-1142, 1117-1143, 1167-1193, 1168-1194, 1211-1237, 1225-1251, 1229-1255, 1238-1264, 1252-1278, 1258-1284, 1260-1286, 1262-1288, 1326-1352, 1328-1354, 1334-1360, 1489-1515, 1493-1519, 1494-1520, 1495-1521, 1497-1523, 1777-1803, 1778-1804, 2264-2290, 2786-2812, or 2787-2813 of the nucleotide sequence set forth in SEQ ID NO: 1.

In some embodiments, the target region is positions 605-625, 607-627, 608-628, 796-816, 797-817, 798-818, 799-819, 800-820, 802-822, 836-856, 1110-1130, 1111-1131, 1119-1139, 1120-1140, 1170-1190, 1171-1191, 1214-1234, 1228-1248, 1232-1252, 1241-1261, 1255-1275, 1261-1281, 1263-1283, 1265-1285, 1329-1349, 1331-1351, 1337-1357, 1492-1512, 1496-1516, 1497-1517, 1498-1518, 1500-1520, 1780-1800, 1781-1801, 2267-2287, 2789-2809, or 2790-2810 of the nucleotide sequence set forth in SEQ ID NO: 1.

In some embodiments, the antisense strand comprises at least 15 contiguous nucleotides of a nucleotide sequence selected from SEQ ID NOs: 40 to 76 and nucleotide sequences having 1 to 3 nucleotide differences therefrom.

In some embodiments, the duplex region is 17 to 23 base pairs in length, preferably 18 to 21 base pairs, more preferably 19 base pairs.

In some embodiments, each of the sense strand and the antisense strand is 17 to 23 nucleotides in length, preferably 19 to 21 nucleotides.

In some embodiments, the RNAi agent comprises one or two blunt ends, preferably one blunt end.

In some embodiments, the RNAi agent comprises one or two overhangs, preferably one overhang, each overhang comprising 1 to 4 unpaired nucleotides, preferably 2 unpaired nucleotides.

In some embodiments, the overhang is located at 3'-end of the sense strand, 3'-end of the antisense strand, or simultaneously at 3'-end of the sense strand and 3'-end of the antisense strand; preferably, the overhang is located at 3'-end of the antisense strand, and more preferably, the RNAi agent has one blunt end.

In some embodiments, the sense strand comprises at least 15 contiguous nucleotides of any nucleotide sequence selected from SEQ ID NOs: 3 to 39 and nucleotide sequences having 1 to 3 nucleotide differences therefrom.

In some embodiments, the antisense strand has no more than 23 nucleotides and comprises a nucleotide sequence selected from SEQ ID NOs: 40 to 76; and the sense strand has no more than 21 nucleotides and comprises a nucleotide sequence from SEQ ID NOs: 3 to 39.

In some embodiments, in the RNAi agent:
the sense strand comprises or consists of a sequence set forth in SEQ ID NO:3 or a nucleotide sequence having 1, 2, or 3 nucleotide differences therefrom, and the antisense strand comprises or consists of a sequence set forth in SEQ ID NO:40 or a nucleotide sequence having 1, 2, or 3 nucleotide differences therefrom;
the sense strand comprises or consists of a sequence set forth in SEQ ID NO:4 or a nucleotide sequence having 1, 2, or 3 nucleotide differences therefrom, and the antisense strand comprises or consists of a sequence set forth in SEQ ID NO:41 or a nucleotide sequence having 1, 2, or 3 nucleotide differences therefrom;
the sense strand comprises or consists of a sequence set forth in SEQ ID NO:5 or a nucleotide sequence having 1, 2, or 3 nucleotide differences therefrom, and the antisense strand comprises or consists of a sequence set forth in SEQ ID NO:42 or a nucleotide sequence having 1, 2, or 3 nucleotide differences therefrom;
the sense strand comprises or consists of a sequence set forth in SEQ ID NO:6 or a nucleotide sequence having 1, 2, or 3 nucleotide differences therefrom, and the antisense strand comprises or consists of a sequence set forth in SEQ ID NO:43 or a nucleotide sequence having 1, 2, or 3 nucleotide differences therefrom;
the sense strand comprises or consists of a sequence set forth in SEQ ID NO:7 or a nucleotide sequence having 1, 2, or 3 nucleotide differences therefrom, and the antisense strand comprises or consists of a sequence set forth in SEQ ID NO:44 or a nucleotide sequence having 1, 2, or 3 nucleotide differences therefrom;
the sense strand comprises or consists of a sequence set forth in SEQ ID NO:8 or a nucleotide sequence having 1, 2, or 3 nucleotide differences therefrom, and the antisense strand comprises or consists of a sequence set forth in SEQ ID NO:45 or a nucleotide sequence having 1, 2, or 3 nucleotide differences therefrom;
the sense strand comprises or consists of a sequence set forth in SEQ ID NO:9 or a nucleotide sequence having 1, 2, or 3 nucleotide differences therefrom, and the antisense strand comprises or consists of a sequence set forth in SEQ ID NO:46 or a nucleotide sequence having 1, 2, or 3 nucleotide differences therefrom;
the sense strand comprises or consists of a sequence set forth in SEQ ID NO:10 or a nucleotide sequence having 1, 2, or 3 nucleotide differences therefrom, and the antisense strand comprises or consists of a sequence set forth in SEQ ID NO:47 or a nucleotide sequence having 1, 2, or 3 nucleotide differences therefrom;
the sense strand comprises or consists of a sequence set forth in SEQ ID NO:11 or a nucleotide sequence having 1, 2, or 3 nucleotide differences therefrom, and the antisense strand comprises or consists of a sequence set forth in SEQ ID NO:48 or a nucleotide sequence having 1, 2, or 3 nucleotide differences therefrom;
the sense strand comprises or consists of a sequence set forth in SEQ ID NO:12 or a nucleotide sequence having 1, 2, or 3 nucleotide differences therefrom, and the antisense strand comprises or consists of a sequence set forth in SEQ ID NO:49 or a nucleotide sequence having 1, 2, or 3 nucleotide differences therefrom;
the sense strand comprises or consists of a sequence set forth in SEQ ID NO:13 or a nucleotide sequence having 1, 2, or 3 nucleotide differences therefrom, and the antisense strand comprises or consists of a sequence set forth in SEQ ID NO:50 or a nucleotide sequence having 1, 2, or 3 nucleotide differences therefrom;
the sense strand comprises or consists of a sequence set forth in SEQ ID NO:14 or a nucleotide sequence having 1, 2, or 3 nucleotide differences therefrom, and the antisense strand comprises or consists of a sequence set forth in SEQ ID NO:51 or a nucleotide sequence having 1, 2, or 3 nucleotide differences therefrom;
the sense strand comprises or consists of a sequence set forth in SEQ ID NO:15 or a nucleotide sequence having 1, 2, or 3 nucleotide differences therefrom, and the antisense strand comprises or consists of a sequence set forth in SEQ ID NO:52 or a nucleotide sequence having 1, 2, or 3 nucleotide differences therefrom;
the sense strand comprises or consists of a sequence set forth in SEQ ID NO:16 or a nucleotide sequence having 1, 2, or 3 nucleotide differences therefrom, and the antisense strand comprises or consists of a sequence set forth in SEQ ID NO:53or a nucleotide sequence having 1, 2, or 3 nucleotide differences therefrom;
the sense strand comprises or consists of a sequence set forth in SEQ ID NO:17 or a nucleotide sequence having 1, 2, or 3 nucleotide differences therefrom, and the antisense strand comprises or consists of a sequence set forth in SEQ ID NO:54or a nucleotide sequence having 1, 2, or 3 nucleotide differences therefrom;
the sense strand comprises or consists of a sequence set forth in SEQ ID NO:18 or a nucleotide sequence having 1, 2, or 3 nucleotide differences therefrom, and the antisense strand comprises or consists of a sequence set forth in SEQ ID NO:55 or a nucleotide sequence having 1, 2, or 3 nucleotide differences therefrom;
the sense strand comprises or consists of a sequence set forth in SEQ ID NO:19 or a nucleotide sequence having 1, 2, or 3 nucleotide differences therefrom, and the antisense strand comprises or consists of a sequence set forth in SEQ ID NO:56 or a nucleotide sequence having 1, 2, or 3 nucleotide differences therefrom;
the sense strand comprises or consists of a sequence set forth in SEQ ID NO:20 or a nucleotide sequence having 1, 2, or 3 nucleotide differences therefrom, and the antisense strand comprises or consists of a sequence set forth in SEQ ID NO:57 or a nucleotide sequence having 1, 2, or 3 nucleotide differences therefrom;
the sense strand comprises or consists of a sequence set forth in SEQ ID NO:21 or a nucleotide sequence having 1, 2, or 3 nucleotide differences therefrom, and the antisense strand comprises or consists of a sequence set forth in SEQ ID NO:58 or a nucleotide sequence having 1, 2, or 3 nucleotide differences therefrom;
the sense strand comprises or consists of a sequence set forth in SEQ ID NO:22 or a nucleotide sequence having 1, 2, or 3 nucleotide differences therefrom, and the antisense strand comprises or consists of a sequence set forth in SEQ ID NO:59 or a nucleotide sequence having 1, 2, or 3 nucleotide differences therefrom;
the sense strand comprises or consists of a sequence set forth in SEQ ID NO:23 or a nucleotide sequence having 1, 2, or 3 nucleotide differences therefrom, and the antisense strand comprises or consists of a sequence set forth in SEQ ID NO:60 or a nucleotide sequence having 1, 2, or 3 nucleotide differences therefrom;
the sense strand comprises or consists of a sequence set forth in SEQ ID NO:24 or a nucleotide sequence having 1, 2, or 3 nucleotide differences therefrom, and the antisense strand comprises or consists of a sequence set forth in SEQ ID NO:61 or a nucleotide sequence having 1, 2, or 3 nucleotide differences therefrom;
the sense strand comprises or consists of a sequence set forth in SEQ ID NO:25 or a nucleotide sequence having 1, 2, or 3 nucleotide differences therefrom, and the antisense strand comprises or consists of a sequence set forth in SEQ ID NO:62 or a nucleotide sequence having 1, 2, or 3 nucleotide differences therefrom;
the sense strand comprises or consists of a sequence set forth in SEQ ID NO:26 or a nucleotide sequence having 1, 2, or 3 nucleotide differences therefrom, and the antisense strand comprises or consists of a sequence set forth in SEQ ID NO:63 or a nucleotide sequence having 1, 2, or 3 nucleotide differences therefrom;
the sense strand comprises or consists of a sequence set forth in SEQ ID NO:27 or a nucleotide sequence having 1, 2, or 3 nucleotide differences therefrom, and the antisense strand comprises or consists of a sequence set forth in SEQ ID NO:64 or a nucleotide sequence having 1, 2, or 3 nucleotide differences therefrom;
the sense strand comprises or consists of a sequence set forth in SEQ ID NO:28 or a nucleotide sequence having 1, 2, or 3 nucleotide differences therefrom, and the antisense strand comprises or consists of a sequence set forth in SEQ ID NO:65 or a nucleotide sequence having 1, 2, or 3 nucleotide differences therefrom;
the sense strand comprises or consists of a sequence set forth in SEQ ID NO:29 or a nucleotide sequence having 1, 2, or 3 nucleotide differences therefrom, and the antisense strand comprises or consists of a sequence set forth in SEQ ID NO:66 or a nucleotide sequence having 1, 2, or 3 nucleotide differences therefrom;
the sense strand comprises or consists of a sequence set forth in SEQ ID NO:30 or a nucleotide sequence having 1, 2, or 3 nucleotide differences therefrom, and the antisense strand comprises or consists of a sequence set forth in SEQ ID NO:67 or a nucleotide sequence having 1, 2, or 3 nucleotide differences therefrom;
the sense strand comprises or consists of a sequence set forth in SEQ ID NO:31 or a nucleotide sequence having 1, 2, or 3 nucleotide differences therefrom, and the antisense strand comprises or consists of a sequence set forth in SEQ ID NO:68 or a nucleotide sequence having 1, 2, or 3 nucleotide differences therefrom;
the sense strand comprises or consists of a sequence set forth in SEQ ID NO:32 or a nucleotide sequence having 1, 2, or 3 nucleotide differences therefrom, and the antisense strand comprises or consists of a sequence set forth in SEQ ID NO:69 or a nucleotide sequence having 1, 2, or 3 nucleotide differences therefrom;
the sense strand comprises or consists of a sequence set forth in SEQ ID NO:33 or a nucleotide sequence having 1, 2, or 3 nucleotide differences therefrom, and the antisense strand comprises or consists of a sequence set forth in SEQ ID NO:70 or a nucleotide sequence having 1, 2, or 3 nucleotide differences therefrom;
the sense strand comprises or consists of a sequence set forth in SEQ ID NO:34 or a nucleotide sequence having 1, 2, or 3 nucleotide differences therefrom, and the antisense strand comprises or consists of a sequence set forth in SEQ ID NO:71 or a nucleotide sequence having 1, 2, or 3 nucleotide differences therefrom;
the sense strand comprises or consists of a sequence set forth in SEQ ID NO:35 or a nucleotide sequence having 1, 2, or 3 nucleotide differences therefrom, and the antisense strand comprises or consists of a sequence set forth in SEQ ID NO:72 or a nucleotide sequence having 1, 2, or 3 nucleotide differences therefrom;
the sense strand comprises or consists of a sequence set forth in SEQ ID NO:36 or a nucleotide sequence having 1, 2, or 3 nucleotide differences therefrom, and the antisense strand comprises or consists of a sequence set forth in SEQ ID NO:73 or a nucleotide sequence having 1, 2, or 3 nucleotide differences therefrom;
the sense strand comprises or consists of a sequence set forth in SEQ ID NO:37 or a nucleotide sequence having 1, 2, or 3 nucleotide differences therefrom, and the antisense strand comprises or consists of a sequence set forth in SEQ ID NO:74 or a nucleotide sequence having 1, 2, or 3 nucleotide differences therefrom;
the sense strand comprises or consists of a sequence set forth in SEQ ID NO:38 or a nucleotide sequence having 1, 2, or 3 nucleotide differences therefrom, and the antisense strand comprises or consists of a sequence set forth in SEQ ID NO:75 or a nucleotide sequence having 1, 2, or 3 nucleotide differences therefrom; or
the sense strand comprises or consists of a sequence set forth in SEQ ID NO:39 or a nucleotide sequence having 1, 2, or 3 nucleotide differences therefrom, and the antisense strand comprises or consists of a sequence set forth in SEQ ID NO:76 or a nucleotide sequence having 1, 2, or 3 nucleotide differences therefrom.

In some embodiments, the RNAi agent comprises duplex 8033, 8034, 8035, 8064, 8065, 8066, 8067, 8068, 8069, 8070, 8088, 8089, 8090, 8091, 8105, 8106, 8108, 8110, 8111, 8112, 8113, 8114, 8115, 8116, 8120, 8121, 8122, 8139, 8140, 8141, 8142, 8143, 8163, 8164, 8290, 8425, or 8426.

In some embodiments, the sense strand and/or the antisense strand of the RNAi agent comprises at least one modified nucleotide, the modified nucleotide independently selected from 2'-deoxy-thymidine (dT) nucleotide, 2'-O-methyl-modified nucleotide (2'-OMe), 2'-fluoro-modified nucleotide (2'-F), 2'-deoxy-modified nucleotide, locked nucleic acid (LNA), unlocked nucleic acid (UNA), bridged nucleic acid (BNA), glycol nucleic acid (GNA), threose nucleic acid (TNA), conformationally restricted nucleotide, constrained ethyl nucleotide (cEt), 2'-amino-modified nucleotide, 2'-O-allyl-modified nucleotide, 2'-C-alkyl-modified nucleotide, 2'-O-methoxyethyl-modified nucleotide (2'-MOE), abasic nucleotide, inverted abasic nucleotide, inverted nucleotide, morpholino nucleotide (MOP), phosphoramidate (PN), tetrahydropyran-modified nucleotide (THP), 1,5-anhydrohexitol-modified nucleotide (HNA), cyclohexenyl-modified nucleotide, nucleotide comprising a phosphorothioate group (PS), nucleotide comprising a methylphosphonate group, nucleotide comprising a 5'-phosphate, nucleotide comprising a 5'-phosphate mimic, cationic lipid-conjugated nucleotide, nucleotide comprising a 5'-vinylphosphonate (5'-VP), and combinations thereof; preferably selected from 2'-O-methyl-modified nucleotide, 2'-fluoro-modified nucleotide, nucleotide comprising a phosphorothioate internucleotide linkage, and combinations thereof; and/or preferably, each nucleotide of the sense strand and/or the antisense strand of the RNAi agent is modified.

In some embodiments, in the RNAi agent, in 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides.

In some embodiments, in the RNAi agent, the antisense strand comprises at least one phosphorothioate internucleotide linkage; preferably, the phosphorothioate internucleotide linkage is present at one or more of the following locations: (i) between nucleotide positions 1 and 2 at 5'-end of the antisense strand; (ii) between nucleotide positions 2 and 3 at 5'-end of the antisense strand; (iii) between nucleotide positions 1 and 2 at 3'-end of the antisense strand; and (iv) between nucleotide positions 2 and 3 at 3'-end of the antisense strand.

In some embodiments, in the RNAi agent, in 5' to 3' direction, the nucleotides at positions 7 and 9 of the sense strand are 2'-fluoro-modified nucleotides, and one or two of the nucleotides at positions 5, 8, and 11 of the sense strand are 2'-fluoro-modified nucleotides, while the nucleotides at remaining positions of the sense strand are all 2'-methoxy-modified nucleotides.

In some embodiments, the sense strand has at least one phosphorothioate internucleoside linkage; preferably, the phosphorothioate internucleoside linkage is present at one or more of the following positions: (i) between the 1st nucleotide and the 2nd nucleotide at the 5' end of the sense strand; (ii) between the 2nd nucleotide and the 3rd nucleotide at the 5' end of the sense strand; (iii) between the 1st nucleotide and the 2nd nucleotide at the 3' end of the sense strand; and (iv) between the 2nd nucleotide and the 3rd nucleotide at the 3' end of the sense strand.

In some embodiments, the modification is selected from one of STC, ESC, Advanced ESC, ESC+, AD1-3, AD5 and GalXC.

In some embodiments, the modification is that the 5'-terminal nucleotide of the antisense strand contains a phosphate or phosphate analog modification, preferably 5'-VP.

In some embodiments, the RNAi agent comprises a ligand targeting hepatocytes, preferably, the ligand comprises a galactose moiety, a galactosamine moiety, or an N-acetylgalactosamine moiety, more preferably, the ligand is a trivalent or tetravalent N-acetylgalactosamine moiety, and even more preferably, the hepatocyte-targeting ligand is L96, NAG25, or NAG37. Alternatively, the RNAi agent comprises a ligand targeting non-hepatocyte cells, preferably a lipophilic group, an integrin ligand, or a transferrin receptor 1 ligand. The lipophilic group is preferably selected from: lipids, vitamins, steroids, C₅-C₃₀ saturated or unsaturated fatty acids, C₅-C₃₀ alkyl groups, and polypeptides comprising at least one positively charged amino acid residue; the lipophilic group is more preferably selected from cholesterol, C₁₆ saturated or unsaturated fatty acid, C₁₆ alkyl group, C₂₂ saturated or unsaturated fatty acid, or C₂₂ alkyl group.

Another aspect of the present invention provides a pharmaceutical composition comprising any one of the RNAi agents of the present invention and a pharmaceutically acceptable carrier; preferably, the pharmaceutical composition is formulated for intravenous or subcutaneous injection.

A further aspect of the present invention provides the use of any RNAi agent of the present invention in the manufacture of a medicament for:
(i) reducing expression level of LPAR1 in a cell;
(ii) preventing or treating a disease mediated by aberrant expression levels of LPAR1; or
(iii) prevention or treatment of a disease or condition selected from fibrotic diseases (e.g., pulmonary fibrosis, hepatic fibrosis, renal fibrosis), hypertrophic cardiomyopathy, diffuse scleroderma, adult-onset scleroderma, cancers (e.g., hepatocellular carcinoma, ovarian cancer, glioblastoma), diabetic nephropathy, atherosclerosis, rheumatoid arthritis, neuropathic pain, non-alcoholic steatohepatitis, benign prostatic hyperplasia, and demyelination.

Yet another aspect of the present invention provides use of an agent that reduces expression of the gene encoding lysophosphatidic acid receptor 1 (LPAR1) in the manufacture of a medicament for the treatment of a disease or condition treatable by LPAR1 knockdown. Preferably, the agent is an RNAi agent. Preferably, the disease or condition is selected from fibrotic diseases (e.g., pulmonary fibrosis, hepatic fibrosis, renal fibrosis), hypertrophic cardiomyopathy, diffuse scleroderma, adult-onset scleroderma, cancers (e.g., hepatocellular carcinoma, ovarian cancer, glioblastoma), diabetic nephropathy, atherosclerosis, rheumatoid arthritis, neuropathic pain, non-alcoholic steatohepatitis, benign prostatic hyperplasia, and demyelination.

Accordingly, the present invention also provides a method of reducing LPAR1 expression level in a cell, the method comprising administering to a subject in need thereof a therapeutically effective amount of any one of the RNAi agents of the present invention. The present invention also provides a method for preventing or treating a disease mediated by aberrant LPAR1 expression level (e.g., caused by elevated levels thereof), the method comprising administering to a subject in need thereof a therapeutically effective amount of any one of the RNAi agents of the present invention. The present invention also provides a method for preventing or treating a disease selected from fibrotic diseases (e.g., pulmonary fibrosis, hepatic fibrosis, renal fibrosis), hypertrophic cardiomyopathy, diffuse scleroderma, adult-onset scleroderma, cancers (e.g., hepatocellular carcinoma, ovarian cancer, glioblastoma), diabetic nephropathy, atherosclerosis, rheumatoid arthritis, neuropathic pain, non-alcoholic steatohepatitis, benign prostatic hyperplasia, and demyelination, the method comprising administering to a subject in need thereof a therapeutically effective amount of any one of the RNAi agents of the present invention. The present invention also provides use of any one of the RNAi agents of the present invention in the manufacture of a medicament for the prevention or treatment of the above-mentioned diseases. The present invention also provides any one of the RNAi agents of the present invention for use in the prevention or treatment of the above-mentioned diseases.

Other aspects of the invention will be apparent from the detailed description of the specification that follows.

### Detailed Description of the Invention

### Definition

Lysophosphatidic acid receptor 1 (LPAR1), as used herein, refers to the lysophosphatidic acid receptor 1 protein or its encoding gene, also known as EDG2, LPA1, VZG1, edg-2, vzg-1, Gpcr26, Mrec1.3, or rec.1.3, with Gene ID: 1902. The NCBI accession number for the full-length human LPAR1 mRNA transcript is NM_001351411.2. The NCBI accession number for the cynomolgus monkey LPAR1 mRNA transcript is XM_005581086.3.

As used herein, "oligonucleotide" refers to a nucleotide sequence that forms a chain of nucleotides via internucleoside linkages, wherein each nucleoside and internucleoside linkage may be modified or unmodified. Unless otherwise indicated, the oligonucleotide consists of 12 to 30 linked nucleotides.

"Internucleoside linkage" refers to a covalent linkage between adjacent nucleotides in an oligonucleotide. As used herein, "modified internucleoside linkage" refers to any internucleoside linkage other than a phosphodiester internucleoside linkage. "Phosphorothioate internucleoside linkage" is a modified internucleoside linkage in which one non-bridging oxygen atom of the phosphodiester internucleoside linkage is replaced by a sulfur atom.

As used herein, the term "RNAi agent" refers to a reagent comprising an RNA molecule that, when introduced into a cell, is capable of down-regulating the expression of a target gene (in this context, the LPAR1 gene) through an RNA interference mechanism. The terms "RNAi agent of the present invention," "RNAi agent described herein," or similar expressions encompass both the modified RNAi agents of the present invention, irrespective of sequence and target gene, and the RNAi agents of the present invention with specific sequences for interfering with the LPAR1 gene. RNA interference refers to a process whereby a nucleic acid molecule induces cleavage and degradation of a target RNA molecule (e.g., an mRNA molecule) in a sequence-specific manner, such as through the RNA-induced silencing complex (RISC) pathway. RNAi agents herein include siRNA, shRNA, and DNA/RNA hybrid molecules, also sometimes collectively referred to as double-stranded RNA (dsRNA), which comprise two antiparallel continuous nucleotide strands sufficiently complementary to hybridize and form a double-stranded region. "Hybridization" refers to the pairing of complementary polynucleotides, typically through hydrogen bonding between complementary bases in the two polynucleotides (e.g., Watson-Crick hydrogen bonding, Wobble hydrogen bonding, Hoogsteen hydrogen bonding, or reverse Hoogsteen hydrogen bonding). The "double-stranded region" refers to an area within two complementary or substantially complementary polynucleotides that form base pairs through hybridization, thereby creating a duplex between the two polynucleotide strands.

The term "antisense strand" refers to the strand in a dsRNA that contains a region substantially complementary to a target sequence. The term "sense strand" or "passenger strand" refers to the strand in a dsRNA that contains a region substantially complementary to the antisense strand region as defined herein. The term "region substantially complementary" denotes a region that is either perfectly complementary or imperfectly complementary. When the complementary region is imperfectly complementary to the target sequence, mismatches may occur in internal or terminal regions of the molecule. Typically, the most tolerated mismatches are located in terminal regions, for example, within 5, 4, 3, or 2 nucleotides at the 5' and/or 3' termini of the dsRNA.

"siRNA" refers to a nucleic acid that forms a double-stranded RNA capable of reducing or inhibiting the expression of a target gene when present in the same cell as the target gene. An siRNA is typically approximately 15 to approximately 30 base pairs in length, most typically approximately 19 to 25 base pairs in length, for example, 19, 20, 21, 22, 23, 24, or 25 nucleotide pairs in length.

"shRNA" refers to short hairpin RNA, which comprises two short inverted repeats and an intervening stem-loop structure connecting them. The stem-loop may comprise at least one unpaired nucleotide, for example, at least 2, at least 3, at least 4, at least 5, at least 6, at least 7, at least 8, at least 9, at least 10, at least 20, at least 23, or more unpaired nucleotides. The stem-loop may be 10 or fewer nucleotides in length. The stem-loop may comprise 8 or fewer unpaired nucleotides. The stem-loop may comprise 4 to 10 unpaired nucleotides. The stem-loop may be 4 to 8 nucleotides in length.

The two substantially complementary strands of a dsRNA need not be, but may be, covalently linked. The maximum number of base pairs is the number of nucleotides in the shortest strand of the dsRNA minus any overhangs present in the duplex. In addition to the duplex structure, a dsRNA may comprise one or more nucleotide overhangs. An overhanging nucleotide refers to one or more unpaired nucleotides extending beyond the double-stranded region at the terminus of a strand. Nucleotide overhangs typically occur when the 3' end of one strand extends beyond the 5' end of the other strand, or when the 5' end of one strand extends beyond the 3' end of the other strand. For example, at least one strand comprises a 3' overhang of at least 1 nucleotide, e.g., 1 to 4 nucleotide overhangs. In another example, at least one strand comprises a 5' overhang of at least 1 nucleotide, e.g., 1 to 4 nucleotide overhangs. In other embodiments, both the 3' terminus and the 5' terminus of one strand of the dsRNA comprise an overhang of at least 1 nucleotide.

As used herein, the terms "blunt end" or "blunt terminus" with respect to dsRNA refer to the absence of unpaired nucleotides or nucleotide analogs at a given terminus of the dsRNA, i.e., no nucleotide overhang. One end or both ends of the dsRNA may be blunt. Where both ends of the dsRNA are blunt, the dsRNA is referred to as blunt-ended. It is to be expressly understood that a "blunt-ended" dsRNA is one having blunt termini at both ends, meaning no nucleotide overhang exists at either end of the molecule. In most cases, such molecules are double-stranded throughout their entire length. As used herein, the term "nucleotide overhang" refers to at least one unpaired nucleotide projecting from the duplex structure of the dsRNA. For example, a nucleotide overhang exists when the 3' terminus of one strand of the dsRNA extends beyond the 5' terminus of the other strand, or vice versa. The overhang may comprise or consist of nucleotides/nucleosides analogs, including deoxyribonucleotides/ deoxyribonucleosides. Overhangs may be present on the sense strand, antisense strand, or any combination thereof. Furthermore, the overhanging nucleotides may reside at the 5' terminus, 3' terminus, or both termini of the antisense strand or sense strand of the dsRNA.

The dsRNA molecule may comprise chemical modifications to ribonucleotides, including modifications to the ribose, base, or backbone components of ribonucleic acid, as described herein or known in the art. Any such modifications, when used in double-stranded ribonucleic acid molecules (e.g., siRNA, shRNA, etc.), are encompassed by the term "dsRNA" for purposes of this disclosure. A "modified" nucleotide refers to a nucleotide that independently has a modified sugar moiety, a modified internucleoside linkage, and/or a modified nucleobase. Accordingly, the term "modified nucleotide" includes substitutions, additions, or removals of functional groups or atoms to the internucleoside linkage, sugar moiety, or nucleobase.

The term "ligand" refers to a cell or tissue targeting agent that binds to a specified cell type (e.g., hepatocytes), such as a lectin, glycoprotein, lipid, or protein (e.g., an antibody). Exemplary targeting agents include thyrotropin, melanotropin, lectins, glycoproteins, surfactant protein A, mucin carbohydrates, multivalent lactose, multivalent galactose, N-acetylgalactosamine (GalNAc), multivalent (e.g., bivalent or trivalent) GalNAc, N-acetylglucosamine, multivalent mannose, multivalent fucose, glycosylated polyamino acids, multivalent galactose, transferrin, bisphosphonates, polyglutamate, polyaspartate, cholesterol, steroids, bile acids, folate, vitamin B12, biotin, RGD peptides, and RGD peptide mimetics. In preferred embodiments, the ligand is a carbohydrate, such as a monosaccharide, disaccharide, trisaccharide, tetrasaccharide, or polysaccharide. For example, the ligand may be a derivative comprising GalNAc. In preferred embodiments, the ligand is a derivative comprising one or more N-acetylgalactosamine moieties attached via a bivalent or trivalent branched linker.

The term "therapeutically effective amount" refers to an amount of an RNAi agent or composition of the present invention that is effective to produce a desired therapeutic effect in at least a subpopulation of cells in an animal at a reasonable benefit/risk ratio applicable to any medical treatment.

The term "pharmaceutically acceptable" refers to compounds, materials, compositions, and/or dosage forms that are, within the scope of sound medical judgment, suitable for contact with tissues of humans and animals without excessive toxicity, irritation, allergic reactions, or other problems or complications and commensurate with a reasonable benefit/risk ratio.

The term "pharmaceutically acceptable carrier" refers to a pharmaceutically acceptable material, composition, or vehicle that participates in carrying or transporting an RNAi agent from one organ or part of the body to another organ or part of the body, such as a liquid or solid filler, diluent, excipient, manufacturing aid, or solvent encapsulating material. Each carrier must be "acceptable" in the sense of being compatible with other ingredients of the composition and innocuous to the patient.

The term "treatment" encompasses prevention, therapy, and cure. The patient receiving such treatment is typically any animal in need, including primates (particularly humans) and other mammals such as horses, cattle, swine, sheep, poultry, and companion animals.

### RNAi Agents For Inhibiting LPAR1 Gene Expression

One aspect of the present invention provides an RNAi agent for inhibiting LPAR1 gene expression, comprising a sense strand and an antisense strand that form a complementary duplex region, wherein the antisense strand comprises at least 15, at least 16, at least 17, at least 18, at least 19, at least 20, or at least 21 contiguous nucleotides that are at least 80%, 85%, 90%, 95%, or 100% complementary to an equal-length contiguous nucleotide sequence of a target region of mRNA encoding LPAR1, or a nucleotide sequence having no more than 3 nucleotide differences therefrom.

In some embodiments, the target region is positions 602-631, 793-825, 833-859, 1107-1143, 1167-1194, 1211-1288, 1326-1360, 1489-1523, 1777-1804, 2264-2290, or 2786-2813 of the nucleotide sequence set forth in SEQ ID NO: 1. In some embodiments, the target region is positions 602-628, 604-630, 605-631, 793-819, 794-820, 795-821, 796-822, 797-823, 799-825, 833-859, 1107-1133, 1108-1134, 1116-1142, 1117-1143, 1167-1193, 1168-1194, 1211-1237, 1225-1251, 1229-1255, 1238-1264, 1252-1278, 1258-1284, 1260-1286, 1262-1288, 1326-1352, 1328-1354, 1334-1360, 1489-1515, 1493-1519, 1494-1520, 1495-1521, 1497-1523, 1777-1803, 1778-1804, 2264-2290, 2786-2812, or 2787-2813 of the nucleotide sequence set forth in SEQ ID NO: 1. In some embodiments, the target region is positions 605-625, 607-627, 608-628, 796-816, 797-817, 798-818, 799-819, 800-820, 802-822, 836-856, 1110-1130, 1111-1131, 1119-1139, 1120-1140, 1170-1190, 1171-1191, 1214-1234, 1228-1248, 1232-1252, 1241-1261, 1255-1275, 1261-1281, 1263-1283, 1265-1285, 1329-1349, 1331-1351, 1337-1357, 1492-1512, 1496-1516, 1497-1517, 1498-1518, 1500-1520, 1780-1800, 1781-1801, 2267-2287, 2789-2809, or 2790-2810 of the nucleotide sequence set forth in SEQ ID NO: 1.

Therefore, in some embodiments, the present invention provides an RNAi agent for inhibiting LPAR1 gene expression, comprising a sense strand and an antisense strand that form a complementary duplex region, wherein the antisense strand comprises at least 15, at least 16, at least 17, at least 18, at least 19, at least 20, or at least 21 contiguous nucleotides that are at least 80%, 85%, 90%, 95%, or 100% complementary to an equal-length contiguous nucleotide sequence within nucleotides 602-631, 793-825, 833-859, 1107-1143, 1167-1194, 1211-1288, 1326-1360, 1489-1523, 1777-1804, 2264-2290, or 2786-2813 of the nucleotide sequence set forth in SEQ ID NO: 1, or a nucleotide sequence having no more than 3 nucleotide differences therefrom.

In preferable embodiments, the present invention provides an RNAi agent for inhibiting LPAR1 gene expression, comprising a sense strand and an antisense strand that form a complementary duplex region, wherein the antisense strand comprises at least 15, at least 16, at least 17, at least 18, at least 19, at least 20, or at least 21 contiguous nucleotides that are at least 80%, 85%, 90%, 95%, or 100% complementary to an equal-length contiguous nucleotide sequence within nucleotides 602-628, 604-630, 605-631, 793-819, 794-820, 795-821, 796-822, 797-823, 799-825, 833-859, 1107-1133, 1108-1134, 1116-1142, 1117-1143, 1167-1193, 1168-1194, 1211-1237, 1225-1251, 1229-1255, 1238-1264, 1252-1278, 1258-1284, 1260-1286, 1262-1288, 1326-1352, 1328-1354, 1334-1360, 1489-1515, 1493-1519, 1494-1520, 1495-1521, 1497-1523, 1777-1803, 1778-1804, 2264-2290, 2786-2812, or 2787-2813 of the nucleotide sequence set forth in SEQ ID NO: 1, or a nucleotide sequence having no more than 3 nucleotide differences therefrom.

In preferable embodiments, the present invention provides an RNAi agent for inhibiting LPAR1 gene expression, comprising a sense strand and an antisense strand that form a complementary duplex region, wherein the antisense strand comprises at least 15, at least 16, at least 17, at least 18, at least 19, at least 20, or at least 21 contiguous nucleotides that are at least 80%, 85%, 90%, 95%, or 100% complementary to an equal-length contiguous nucleotide sequence within nucleotides 605-625, 607-627, 608-628, 796-816, 797-817, 798-818, 799-819, 800-820, 802-822, 836-856, 1110-1130, 1111-1131, 1119-1139, 1120-1140, 1170-1190, 1171-1191, 1214-1234, 1228-1248, 1232-1252, 1241-1261, 1255-1275, 1261-1281, 1263-1283, 1265-1285, 1329-1349, 1331-1351, 1337-1357, 1492-1512, 1496-1516, 1497-1517, 1498-1518, 1500-1520, 1780-1800, 1781-1801, 2267-2287, 2789-2809, or 2790-2810 of the nucleotide sequence set forth in SEQ ID NO: 1, or a nucleotide sequence having no more than 3 nucleotide differences therefrom.

In some embodiments, the antisense strand comprises at least 15 (e.g., 15, 16, 17, 18, 19, 20, or 21) contiguous nucleotides of a nucleotide sequence selected from SEQ ID NOs: 40 to 76 and nucleotide sequences having 1, 2, or 3 nucleotide differences each therefrom.

In some embodiments, the present invention provides an RNAi agent for inhibiting LPAR1 gene expression, comprising a sense strand and an antisense strand that form a complementary duplex region, wherein the antisense strand comprises at least 15 (e.g., 15, 16, 17, 18, 19, 20, or 21) contiguous nucleotides selected from any one of the nucleotide sequences of SEQ ID NOs: 40 to 76.

In some embodiments, the present invention provides an RNAi agent for inhibiting LPAR1 gene expression, comprising a sense strand and an antisense strand that form a complementary duplex region, wherein the antisense strand comprises at least 15 (e.g., 15, 16, 17, 18, 19, 20, or 21) contiguous nucleotides selected from a nucleotide sequence that differs from any one of the nucleotide sequences of SEQ ID NOs: 40 to 76 by 1, 2, or 3 nucleotides.

In the present invention, when referring to 1, 2, or 3 nucleotide differences, the 1, 2, or 3 nucleotide differences may be on the sense strand and/or antisense strand outside the duplex region. In some embodiments, the 1, 2, or 3 nucleotide differences are on the sense strand and/or antisense strand within the duplex region. In some embodiments, a portion of the 1, 2, or 3 nucleotide differences are on the sense strand and/or antisense strand within the duplex region, and another portion are on the sense strand and/or antisense strand outside the duplex region. For the antisense strand, in some embodiments, the 1, 2, or 3 nucleotide differences are located at the extreme 3' end or extreme 5' end of the antisense strand. In some embodiments, the 1, 2, or 3 nucleotide differences are located between the extreme 3' end and extreme 5' end of the antisense strand. In some embodiments, 1 or 2 of the 1, 2, or 3 nucleotide differences are located at the extreme 3' end or extreme 5' end of the antisense strand, and the other 1 or 2 are located between the extreme 3' end and extreme 5' end of the antisense strand. For the sense strand, in some embodiments, the 1, 2, or 3 nucleotide differences are located at the extreme 3' end or extreme 5' end of the sense strand. In some embodiments, the 1, 2, or 3 nucleotide differences are located between the extreme 3' end and extreme 5' end of the sense strand. In some embodiments, 1 or 2 of the 1, 2, or 3 nucleotide differences are located at the extreme 3' end or extreme 5' end of the sense strand, and the other 1 or 2 are located between the extreme 3' end and extreme 5' end of the sense strand.

In some embodiments, the present invention provides an RNAi agent for inhibiting LPAR1 gene expression, comprising a sense strand and an antisense strand that form a complementary duplex region, wherein the antisense strand comprises at least 15 (e.g., 15, 16, 17, 18, 19, 20, or 21) contiguous nucleotides of a nucleotide sequence having 1 nucleotide difference from any nucleotide sequence selected from SEQ ID NOs: 40 to 76, the 1 nucleotide difference being located at the extreme 3' end of the antisense strand. In some embodiments, the present invention provides an RNAi agent for inhibiting LPAR1 gene expression, comprising a sense strand and an antisense strand that form a complementary duplex region, wherein the antisense strand comprises at least 15 (e.g., 15, 16, 17, 18, 19, 20, or 21) contiguous nucleotides of a nucleotide sequence having 2 nucleotide differences from any nucleotide sequence selected from SEQ ID NOs: 40 to 76, the 2 nucleotide differences being located consecutively at the extreme 3' end of the antisense strand.

In some embodiments, the present invention provides an RNAi agent for inhibiting LPAR1 gene expression, comprising a sense strand and an antisense strand that form a complementary duplex region, wherein the antisense strand comprises no more than 23 nucleotides in length and comprises at least 15 (e.g., 15, 16, 17, 18, 19, 20, or 21) contiguous nucleotides of any nucleotide sequence selected from SEQ ID NOs: 40 to 76.

In some embodiments, the present invention provides an RNAi agent for inhibiting LPAR1 gene expression, comprising a sense strand and an antisense strand that form a complementary duplex region, wherein the antisense strand comprises no more than 23 nucleotides in length and comprises at least 15 (e.g., 15, 16, 17, 18, 19, 20, or 21) contiguous nucleotides of a nucleotide sequence having 1, 2, or 3 nucleotide differences from any nucleotide sequence selected from SEQ ID NOs: 40 to 76.

One aspect of the present invention provides an RNAi agent for inhibiting LPAR1 gene expression, comprising an antisense strand, wherein the antisense strand comprises at least 15 (e.g., 15, 16, 17, 18, 19, 20, or 21) contiguous nucleotides of a nucleotide sequence selected from SEQ ID NOs: 40 to 76 and nucleotide sequences having 1, 2, or 3 nucleotide differences therefrom.

In some embodiments, the present invention provides an RNAi agent for inhibiting LPAR1 gene expression, comprising an antisense strand, wherein the antisense strand comprises at least 15 (e.g., 15, 16, 17, 18, 19, 20, or 21) contiguous nucleotides of any nucleotide sequence selected from SEQ ID NOs: 40 to 76.

In some embodiments, the present invention provides an RNAi agent for inhibiting LPAR1 gene expression, comprising an antisense strand, wherein the antisense strand comprises at least 15 (e.g., 15, 16, 17, 18, 19, 20, or 21) contiguous nucleotides of a nucleotide sequence having 1, 2, or 3 nucleotide differences from any nucleotide sequence selected from SEQ ID NOs: 40 to 76.

In some embodiments, the length of the duplex region is about 17 to about 23 base pairs. For example, suitable lengths for the duplex region are about 17 to about 22 base pairs, about 17 to about 21 base pairs, about 17 to about 20 base pairs, about 17 to about 19 base pairs, about 17 to about 18 base pairs, about 18 to about 23 base pairs, about 18 to about 22 base pairs, about 18 to about 21 base pairs, about 18 to about 20 base pairs, about 19 to 23 base pairs, about 19 to 22 base pairs, about 19 to 21 base pairs, about 20 to 23 base pairs, about 20 to 22 base pairs, or about 21 to 23 base pairs. In certain embodiments, the length of the duplex region is about 18 to about 21 base pairs. In other embodiments, the length of the duplex region is about 19 base pairs.

Accordingly, in some embodiments of the present invention, an RNAi agent for inhibiting LPAR1 gene expression is provided, comprising a sense strand and an antisense strand that form a complementary duplex region, the length of the duplex region being about 17 to about 23 base pairs, and wherein the antisense strand is no more than 23 nucleotides in length and comprises at least 15 contiguous nucleotides of a nucleotide sequence selected from SEQ ID NOs: 40 to 76 and nucleotide sequences having 1 to 3 nucleotide differences therefrom.

In some embodiments of the present invention, an RNAi agent for inhibiting LPAR1 gene expression is provided, comprising a sense strand and an antisense strand that form a complementary duplex region, the length of the duplex region being about 18 to about 21 base pairs, and wherein the antisense strand is no more than 23 nucleotides in length and comprises at least 15 contiguous nucleotides of a nucleotide sequence selected from SEQ ID NOs: 40 to 76 and nucleotide sequences having 1 to 3 nucleotide differences therefrom.

In some embodiments of the present invention, an RNAi agent for inhibiting LPAR1 gene expression is provided, comprising a sense strand and an antisense strand that form a complementary duplex region, the length of the duplex region being about 19 base pairs, and wherein the antisense strand is no more than 23 nucleotides in length and comprises at least 15 (e.g., 15, 16, 17, 18, 19, 20, or 21) contiguous nucleotides of a nucleotide sequence selected from SEQ ID NOs: 40 to 76 and nucleotide sequences having 1, 2, or 3 nucleotide differences therefrom.

In some embodiments, the sense and antisense strands of the RNAi agent of the invention each independently have a length of about 17 to about 23 nucleotides, e.g., about 18 to about 23 nucleotides, about 19 to about 23 nucleotides, about 20 to about 23 nucleotides, about 21 to about 23 nucleotides, about 17 to about 22 nucleotides, about 17 to about 21 nucleotides, about 17 to about 20 nucleotides, about 17 to about 19 nucleotides, about 18 to about 22 nucleotides, about 18 to about 21 nucleotides, about 18 to about 20 nucleotides, about 19 to about 22 nucleotides, about 19 to about 21 nucleotides, or about 20 to about 22 nucleotides. In certain embodiments, the sense and antisense strands each independently have a length of about 17, about 18, about 19, about 20, about 21, about 22, or about 23 nucleotides.

In some embodiments, the sense strand and antisense strand are of identical length but form a duplex region shorter than the full length of the strands, such that the RNAi agent for inhibiting LPAR1 gene expression comprises two nucleotide overhangs. For example, in one embodiment, the RNAi agent for inhibiting LPAR1 gene expression comprises (i) a sense strand and an antisense strand each having a length of 21 nucleotides, (ii) a duplex region having a length of 19 base pairs, and (iii) a single-nucleotide overhang at each of 3'-end of the sense strand and 3'-end of the antisense strand. In another embodiment, the RNAi agent for inhibiting LPAR1 gene expression comprises (i) a sense strand and an antisense strand each having a length of 23 nucleotides, (ii) a duplex region having a length of 21 base pairs, and (iii) a single-nucleotide overhang at each of 3'-end of the sense strand and 3'-end of the antisense strand.

In other embodiments, the sense strand and antisense strand are of identical length and form a duplex region along their entire length, such that no nucleotide overhangs are present at either end of the duplex molecule. In one such embodiment, the RNAi agent for inhibiting LPAR1 gene expression is blunt-ended and comprises (i) a sense strand and an antisense strand each having a length of 21 nucleotides, and (ii) a duplex region having a length of 21 base pairs. In another such embodiment, the RNAi agent for inhibiting LPAR1 gene expression is blunt-ended and comprises (i) a sense strand and an antisense strand each having a length of 23 nucleotides, and (ii) a duplex region having a length of 23 base pairs. In another such embodiment, the RNAi agent for inhibiting LPAR1 gene expression is blunt-ended and comprises (i) a sense strand and an antisense strand each having a length of 19 nucleotides, and (ii) a duplex region having a length of 19 base pairs.

In other embodiments, the sense strand or antisense strand is longer than the other strand, and the two strands form a duplex region of length equal to that of the shorter strand, such that the RNAi agent for inhibiting LPAR1 gene expression comprises at least one nucleotide overhang. For example, in some embodiments, the sense strand is 1 to 4 nucleotides longer than the antisense strand, and the duplex region formed by both strands equals the length of the antisense strand, such that the sense strand forms an overhang comprising 1 to 4 unpaired nucleotides. In other embodiments, the antisense strand is 1 to 4 nucleotides longer than the sense strand, and the duplex region formed by both strands equals the length of the sense strand, such that the antisense strand forms an overhang comprising 1 to 4 unpaired nucleotides. In some embodiments, the nucleotide overhang has a length of 1, 2, 3, or 4 nucleotides. In a specific embodiment, the overhang comprises 2 nucleotides. In certain embodiments, the overhang comprises a single nucleotide.

The overhanging nucleotides may be ribonucleotides or modified nucleotides as described herein. In some embodiments, the overhanging nucleotides are 2'-modified nucleotides (e.g., 2'-fluoro-modified nucleotides, 2'-O-methyl-modified nucleotides) or combinations thereof. For example, in one embodiment, the overhanging nucleotides are deoxyribonucleotides, such as deoxythymidine. In another embodiment, the overhanging nucleotides are selected from: 2'-O-methyl-modified nucleotides, 2'-fluoro-modified nucleotides, 2'-methoxyethyl-modified nucleotides, abasic nucleotides, inverted abasic nucleotides, inverted nucleotides, or combinations thereof. In other embodiments, the overhang comprises a 5'-uridine-uridine-3' (5'-UU-3') dinucleotide. In such embodiments, the UU dinucleotide may comprise ribonucleotides or modified nucleotides, such as 2'-modified nucleotides. In other embodiments, the overhang comprises a 5'-deoxythymidine-deoxythymidine-3' (5'-dTdT-3') dinucleotide. When a nucleotide overhang is present on the antisense strand, nucleotides in the overhang may be complementary to the target gene sequence, may form mismatches with the target gene sequence, or may comprise other sequences (such as UU, TT, AA, GG, etc.).

The nucleotide overhang may be located at the 5'-end or 3'-end of either strand or both strands. For example, in one embodiment, the RNAi agent for inhibiting LPAR1 gene expression comprises nucleotide overhangs at both 5'-end and 3'-end of the antisense strand. In another embodiment, the RNAi agent for inhibiting LPAR1 gene expression comprises nucleotide overhangs at both 5'-end and 3'-end of the sense strand. In some embodiments, the RNAi agent for inhibiting LPAR1 gene expression comprises nucleotide overhangs at the 5'-end of the sense strand and the 5'-end of the antisense strand. In other embodiments, the RNAi agent for inhibiting LPAR1 gene expression comprises nucleotide overhangs at the 3'-end of the sense strand and the 3'-end of the antisense strand. In some embodiments, the RNAi agent for inhibiting LPAR1 gene expression comprises only a nucleotide overhang at the 5'-end of the sense strand. In some embodiments, the RNAi agent for inhibiting LPAR1 gene expression comprises only a nucleotide overhang at the 3'-end of the sense strand. In some embodiments, the RNAi agent for inhibiting LPAR1 gene expression comprises only a nucleotide overhang at the 3'-end of the antisense strand. In some embodiments, the RNAi agent for inhibiting LPAR1 gene expression comprises only a nucleotide overhang at the 5'-end of the antisense strand. In some embodiments, the RNAi agent for inhibiting LPAR1 gene expression comprises only a nucleotide overhang at the 5'-end of the sense strand.

The RNAi agent for inhibiting LPAR1 gene expression may comprise a nucleotide overhang at one end and a blunt end at the opposite end of the double-stranded RNA molecule. "Blunt end" means that the sense strand and antisense strand are fully base-paired at the molecular terminus, with no unpaired nucleotides extending beyond the duplex region. In some embodiments, the RNAi agent for inhibiting LPAR1 gene expression comprises a nucleotide overhang at the 3'-end of the sense strand, and blunt ends at the 5'-end of the sense strand and the 3'-end of the antisense strand. In other embodiments, the RNAi agent for inhibiting LPAR1 gene expression comprises a nucleotide overhang at the 3'-end of the antisense strand, and blunt ends at the 5'-end of the antisense strand and the 3'-end of the sense strand.

Specifically, by way of example, in one embodiment, the RNAi agent for inhibiting LPAR1 gene expression comprises: (i) a sense strand of 19 nucleotides in length, (ii) an antisense strand of 21 nucleotides in length, wherein the strands form a duplex region equal to the length of the sense strand. In another embodiment, the RNAi agent for inhibiting LPAR1 gene expression comprises: (i) a sense strand of 21 nucleotides in length, (ii) an antisense strand of 23 nucleotides in length, wherein the strands form a duplex region equal to the length of the sense strand.

In some embodiments, an RNAi agent for inhibiting LPAR1 gene expression in cells is provided, comprising a sense strand and an antisense strand that form a duplex region, wherein the antisense strand is no more than 23 nucleotides in length and comprises at least 15 (e.g., 15, 16, 17, 18, 19, 20, or 21) contiguous nucleotides of a nucleotide sequence selected from SEQ ID NOs: 40 to 76 and nucleotide sequences having 1, 2, or 3 nucleotide differences therefrom, and wherein the RNAi agent comprises one overhang and one blunt end, the overhang preferably having 2 unpaired nucleotides.

In some embodiments, an RNAi agent for inhibiting LPAR1 gene expression in cells is provided, comprising a sense strand and an antisense strand that form a duplex region, wherein the antisense strand is no more than 23 nucleotides in length and comprises at least 15 (e.g., 15, 16, 17, 18, 19, 20, or 21) contiguous nucleotides of a nucleotide sequence selected from SEQ ID NOs: 40 to 76 and nucleotide sequences having 1, 2, or 3 nucleotide differences therefrom, and wherein the RNAi agent comprises one overhang and one blunt end, the overhang preferably having 2 unpaired nucleotides, wherein the overhang is formed at 3'-end of the antisense strand, and the blunt end is formed at 3'-end of the sense strand and 5'-end of the antisense strand.

In some embodiments, an RNAi agent for inhibiting LPAR1 gene expression in cells is provided, comprising a sense strand and an antisense strand that form a duplex region of 19 base pairs in length, wherein the antisense strand is no more than 23 nucleotides in length and comprises at least 15 (e.g., 15, 16, 17, 18, 19, 20, or 21) contiguous nucleotides of a nucleotide sequence selected from SEQ ID NOs: 40 to 76 and nucleotide sequences having 1, 2, or 3 nucleotide differences therefrom, and wherein the RNAi agent comprises one overhang and one blunt end, the overhang preferably having 2 unpaired nucleotides, wherein the overhang is formed at 3'-end of the antisense strand, and the blunt end is formed at 3'-end of the sense strand and 5'-end of the antisense strand.

In some embodiments, an RNAi agent for inhibiting LPAR1 gene expression in cells is provided, comprising a sense strand and an antisense strand that form a duplex region of 19 base pairs in length, wherein the antisense strand is no more than 23 nucleotides in length and comprises at least 15 (e.g., 15, 16, 17, 18, 19, 20, or 21) contiguous nucleotides of a nucleotide sequence selected from SEQ ID NOs: 40 to 76 and nucleotide sequences having 1, 2, or 3 nucleotide differences therefrom, wherein the sense strand is 19 to 21 nucleotides in length, and wherein the RNAi agent comprises one overhang and one blunt end, the overhang preferably having 2 unpaired nucleotides, wherein the overhang is formed at 3'-end of the antisense strand, and the blunt end is formed at 3'-end of the sense strand and 5'-end of the antisense strand.

In some embodiments, an RNAi agent for inhibiting LPAR1 gene expression in cells is provided, comprising a sense strand and an antisense strand that form a duplex region of 19 base pairs in length, wherein the antisense strand is 21 nucleotides in length and consists of at least 15 (e.g., 15, 16, 17, 18, 19, 20, or 21) contiguous nucleotides of a nucleotide sequence selected from SEQ ID NOs: 40 to 76 and nucleotide sequences having 1, 2, or 3 nucleotide differences therefrom, wherein the sense strand is 19 nucleotides in length, and wherein the RNAi agent comprises one overhang and one blunt end, the overhang preferably having 2 unpaired nucleotides, wherein the overhang is formed at 3'-end of the antisense strand, and the blunt end is formed at 3'-end of the sense strand and 5'-end of the antisense strand.

In some embodiments, the present invention provides an RNAi agent for inhibiting LPAR1 gene expression in cells, comprising a sense strand and an antisense strand that form a duplex region of 19 base pairs in length, wherein the antisense strand is 21 nucleotides in length and consists of any nucleotide sequence selected from SEQ ID NOs: 40 to 76, wherein the sense strand is 19 nucleotides in length, and wherein the RNAi agent comprises one overhang and one blunt end, the overhang preferably having 2 unpaired nucleotides, wherein the overhang is formed at 3'-end of the antisense strand, and the blunt end is formed at 3'-end of the sense strand and 5'-end of the antisense strand.

In preferred embodiments, the present invention provides an RNAi agent for inhibiting LPAR1 gene expression in cells, comprising a sense strand and an antisense strand that form a duplex region, wherein the antisense strand comprises at least 15 (e.g., 15, 16, 17, 18, 19, 20, or 21) contiguous nucleotides of a nucleotide sequence selected from SEQ ID NOs: 40 to 76 and nucleotide sequences having 1, 2, or 3 nucleotide differences therefrom, and wherein the sense strand comprises at least 15 (e.g., 15, 16, 17, 18, or 19) contiguous nucleotides of any nucleotide sequence selected from SEQ ID NOs: 3 to 39 and nucleotide sequences having 1, 2, or 3 nucleotide differences therefrom.

In preferred embodiments, the present invention provides an RNAi agent for inhibiting LPAR1 gene expression in cells, comprising a sense strand and an antisense strand that form a duplex region, wherein the antisense strand is no more than 23 nucleotides in length and comprises at least 15 (e.g., 15, 16, 17, 18, 19, 20, or 21) contiguous nucleotides of a nucleotide sequence selected from SEQ ID NOs: 40 to 76 and nucleotide sequences having 1, 2, or 3 nucleotide differences therefrom, and wherein the sense strand is no more than 21 nucleotides in length and comprises at least 15 (e.g., 15, 16, 17, 18, or 19) contiguous nucleotides of any nucleotide sequence selected from SEQ ID NOs: 3 to 39 and nucleotide sequences having 1, 2, or 3 nucleotide differences therefrom.

In preferred embodiments, the present invention provides an RNAi agent for inhibiting LPAR1 gene expression in cells, comprising a sense strand and an antisense strand that form a duplex region, wherein the antisense strand is no more than 23 nucleotides in length and comprises any nucleotide sequence selected from SEQ ID NOs: 40 to 76, and wherein the sense strand is no more than 21 nucleotides in length and comprises any nucleotide sequence selected from SEQ ID NOs: 3 to 39.

In preferred embodiments, the present invention provides an RNAi agent for inhibiting LPAR1 gene expression in a cell, comprising a sense strand and an antisense strand that form a duplex region, wherein:
the sense strand comprises or consists of a sequence set forth in SEQ ID NO:3 or a nucleotide sequence having 1, 2, or 3 nucleotide differences therefrom, and the antisense strand comprises or consists of a sequence set forth in SEQ ID NO:40 or a nucleotide sequence having 1, 2, or 3 nucleotide differences therefrom;
the sense strand comprises or consists of a sequence set forth in SEQ ID NO:4 or a nucleotide sequence having 1, 2, or 3 nucleotide differences therefrom, and the antisense strand comprises or consists of a sequence set forth in SEQ ID NO:41 or a nucleotide sequence having 1, 2, or 3 nucleotide differences therefrom;
the sense strand comprises or consists of a sequence set forth in SEQ ID NO:5 or a nucleotide sequence having 1, 2, or 3 nucleotide differences therefrom, and the antisense strand comprises or consists of a sequence set forth in SEQ ID NO:42 or a nucleotide sequence having 1, 2, or 3 nucleotide differences therefrom;
the sense strand comprises or consists of a sequence set forth in SEQ ID NO:6 or a nucleotide sequence having 1, 2, or 3 nucleotide differences therefrom, and the antisense strand comprises or consists of a sequence set forth in SEQ ID NO:43 or a nucleotide sequence having 1, 2, or 3 nucleotide differences therefrom;
the sense strand comprises or consists of a sequence set forth in SEQ ID NO:7 or a nucleotide sequence having 1, 2, or 3 nucleotide differences therefrom, and the antisense strand comprises or consists of a sequence set forth in SEQ ID NO:44 or a nucleotide sequence having 1, 2, or 3 nucleotide differences therefrom;
the sense strand comprises or consists of a sequence set forth in SEQ ID NO:8 or a nucleotide sequence having 1, 2, or 3 nucleotide differences therefrom, and the antisense strand comprises or consists of a sequence set forth in SEQ ID NO:45 or a nucleotide sequence having 1, 2, or 3 nucleotide differences therefrom;
the sense strand comprises or consists of a sequence set forth in SEQ ID NO:9 or a nucleotide sequence having 1, 2, or 3 nucleotide differences therefrom, and the antisense strand comprises or consists of a sequence set forth in SEQ ID NO:46 or a nucleotide sequence having 1, 2, or 3 nucleotide differences therefrom;
the sense strand comprises or consists of a sequence set forth in SEQ ID NO:10 or a nucleotide sequence having 1, 2, or 3 nucleotide differences therefrom, and the antisense strand comprises or consists of a sequence set forth in SEQ ID NO:47 or a nucleotide sequence having 1, 2, or 3 nucleotide differences therefrom;
the sense strand comprises or consists of a sequence set forth in SEQ ID NO:11 or a nucleotide sequence having 1, 2, or 3 nucleotide differences therefrom, and the antisense strand comprises or consists of a sequence set forth in SEQ ID NO:48 or a nucleotide sequence having 1, 2, or 3 nucleotide differences therefrom;
the sense strand comprises or consists of a sequence set forth in SEQ ID NO:12 or a nucleotide sequence having 1, 2, or 3 nucleotide differences therefrom, and the antisense strand comprises or consists of a sequence set forth in SEQ ID NO:49 or a nucleotide sequence having 1, 2, or 3 nucleotide differences therefrom;
the sense strand comprises or consists of a sequence set forth in SEQ ID NO:13 or a nucleotide sequence having 1, 2, or 3 nucleotide differences therefrom, and the antisense strand comprises or consists of a sequence set forth in SEQ ID NO:50 or a nucleotide sequence having 1, 2, or 3 nucleotide differences therefrom;
the sense strand comprises or consists of a sequence set forth in SEQ ID NO:14 or a nucleotide sequence having 1, 2, or 3 nucleotide differences therefrom, and the antisense strand comprises or consists of a sequence set forth in SEQ ID NO:51 or a nucleotide sequence having 1, 2, or 3 nucleotide differences therefrom;
the sense strand comprises or consists of a sequence set forth in SEQ ID NO:15 or a nucleotide sequence having 1, 2, or 3 nucleotide differences therefrom, and the antisense strand comprises or consists of a sequence set forth in SEQ ID NO:52 or a nucleotide sequence having 1, 2, or 3 nucleotide differences therefrom;
the sense strand comprises or consists of a sequence set forth in SEQ ID NO:16 or a nucleotide sequence having 1, 2, or 3 nucleotide differences therefrom, and the antisense strand comprises or consists of a sequence set forth in SEQ ID NO:53or a nucleotide sequence having 1, 2, or 3 nucleotide differences therefrom;
the sense strand comprises or consists of a sequence set forth in SEQ ID NO:17 or a nucleotide sequence having 1, 2, or 3 nucleotide differences therefrom, and the antisense strand comprises or consists of a sequence set forth in SEQ ID NO:54or a nucleotide sequence having 1, 2, or 3 nucleotide differences therefrom;
the sense strand comprises or consists of a sequence set forth in SEQ ID NO:18 or a nucleotide sequence having 1, 2, or 3 nucleotide differences therefrom, and the antisense strand comprises or consists of a sequence set forth in SEQ ID NO:55 or a nucleotide sequence having 1, 2, or 3 nucleotide differences therefrom;
the sense strand comprises or consists of a sequence set forth in SEQ ID NO:19 or a nucleotide sequence having 1, 2, or 3 nucleotide differences therefrom, and the antisense strand comprises or consists of a sequence set forth in SEQ ID NO:56 or a nucleotide sequence having 1, 2, or 3 nucleotide differences therefrom;
the sense strand comprises or consists of a sequence set forth in SEQ ID NO:20 or a nucleotide sequence having 1, 2, or 3 nucleotide differences therefrom, and the antisense strand comprises or consists of a sequence set forth in SEQ ID NO:57 or a nucleotide sequence having 1, 2, or 3 nucleotide differences therefrom;
the sense strand comprises or consists of a sequence set forth in SEQ ID NO:21 or a nucleotide sequence having 1, 2, or 3 nucleotide differences therefrom, and the antisense strand comprises or consists of a sequence set forth in SEQ ID NO:58 or a nucleotide sequence having 1, 2, or 3 nucleotide differences therefrom;
the sense strand comprises or consists of a sequence set forth in SEQ ID NO:22 or a nucleotide sequence having 1, 2, or 3 nucleotide differences therefrom, and the antisense strand comprises or consists of a sequence set forth in SEQ ID NO:59 or a nucleotide sequence having 1, 2, or 3 nucleotide differences therefrom;
the sense strand comprises or consists of a sequence set forth in SEQ ID NO:23 or a nucleotide sequence having 1, 2, or 3 nucleotide differences therefrom, and the antisense strand comprises or consists of a sequence set forth in SEQ ID NO:60 or a nucleotide sequence having 1, 2, or 3 nucleotide differences therefrom;
the sense strand comprises or consists of a sequence set forth in SEQ ID NO:24 or a nucleotide sequence having 1, 2, or 3 nucleotide differences therefrom, and the antisense strand comprises or consists of a sequence set forth in SEQ ID NO:61 or a nucleotide sequence having 1, 2, or 3 nucleotide differences therefrom;
the sense strand comprises or consists of a sequence set forth in SEQ ID NO:25 or a nucleotide sequence having 1, 2, or 3 nucleotide differences therefrom, and the antisense strand comprises or consists of a sequence set forth in SEQ ID NO:62 or a nucleotide sequence having 1, 2, or 3 nucleotide differences therefrom;
the sense strand comprises or consists of a sequence set forth in SEQ ID NO:26 or a nucleotide sequence having 1, 2, or 3 nucleotide differences therefrom, and the antisense strand comprises or consists of a sequence set forth in SEQ ID NO:63 or a nucleotide sequence having 1, 2, or 3 nucleotide differences therefrom;
the sense strand comprises or consists of a sequence set forth in SEQ ID NO:27 or a nucleotide sequence having 1, 2, or 3 nucleotide differences therefrom, and the antisense strand comprises or consists of a sequence set forth in SEQ ID NO:64 or a nucleotide sequence having 1, 2, or 3 nucleotide differences therefrom;
the sense strand comprises or consists of a sequence set forth in SEQ ID NO:28 or a nucleotide sequence having 1, 2, or 3 nucleotide differences therefrom, and the antisense strand comprises or consists of a sequence set forth in SEQ ID NO:65 or a nucleotide sequence having 1, 2, or 3 nucleotide differences therefrom;
the sense strand comprises or consists of a sequence set forth in SEQ ID NO:29 or a nucleotide sequence having 1, 2, or 3 nucleotide differences therefrom, and the antisense strand comprises or consists of a sequence set forth in SEQ ID NO:66 or a nucleotide sequence having 1, 2, or 3 nucleotide differences therefrom;
the sense strand comprises or consists of a sequence set forth in SEQ ID NO:30 or a nucleotide sequence having 1, 2, or 3 nucleotide differences therefrom, and the antisense strand comprises or consists of a sequence set forth in SEQ ID NO:67 or a nucleotide sequence having 1, 2, or 3 nucleotide differences therefrom;
the sense strand comprises or consists of a sequence set forth in SEQ ID NO:31 or a nucleotide sequence having 1, 2, or 3 nucleotide differences therefrom, and the antisense strand comprises or consists of a sequence set forth in SEQ ID NO:68 or a nucleotide sequence having 1, 2, or 3 nucleotide differences therefrom;
the sense strand comprises or consists of a sequence set forth in SEQ ID NO:32 or a nucleotide sequence having 1, 2, or 3 nucleotide differences therefrom, and the antisense strand comprises or consists of a sequence set forth in SEQ ID NO:69 or a nucleotide sequence having 1, 2, or 3 nucleotide differences therefrom;
the sense strand comprises or consists of a sequence set forth in SEQ ID NO:33 or a nucleotide sequence having 1, 2, or 3 nucleotide differences therefrom, and the antisense strand comprises or consists of a sequence set forth in SEQ ID NO:70 or a nucleotide sequence having 1, 2, or 3 nucleotide differences therefrom;
the sense strand comprises or consists of a sequence set forth in SEQ ID NO:34 or a nucleotide sequence having 1, 2, or 3 nucleotide differences therefrom, and the antisense strand comprises or consists of a sequence set forth in SEQ ID NO:71 or a nucleotide sequence having 1, 2, or 3 nucleotide differences therefrom;
the sense strand comprises or consists of a sequence set forth in SEQ ID NO:35 or a nucleotide sequence having 1, 2, or 3 nucleotide differences therefrom, and the antisense strand comprises or consists of a sequence set forth in SEQ ID NO:72 or a nucleotide sequence having 1, 2, or 3 nucleotide differences therefrom;
the sense strand comprises or consists of a sequence set forth in SEQ ID NO:36 or a nucleotide sequence having 1, 2, or 3 nucleotide differences therefrom, and the antisense strand comprises or consists of a sequence set forth in SEQ ID NO:73 or a nucleotide sequence having 1, 2, or 3 nucleotide differences therefrom;
the sense strand comprises or consists of a sequence set forth in SEQ ID NO:37 or a nucleotide sequence having 1, 2, or 3 nucleotide differences therefrom, and the antisense strand comprises or consists of a sequence set forth in SEQ ID NO:74 or a nucleotide sequence having 1, 2, or 3 nucleotide differences therefrom;
the sense strand comprises or consists of a sequence set forth in SEQ ID NO:38 or a nucleotide sequence having 1, 2, or 3 nucleotide differences therefrom, and the antisense strand comprises or consists of a sequence set forth in SEQ ID NO:75 or a nucleotide sequence having 1, 2, or 3 nucleotide differences therefrom; or
the sense strand comprises or consists of a sequence set forth in SEQ ID NO:39 or a nucleotide sequence having 1, 2, or 3 nucleotide differences therefrom, and the antisense strand comprises or consists of a sequence set forth in SEQ ID NO:76 or a nucleotide sequence having 1, 2, or 3 nucleotide differences therefrom.

In preferred embodiments, the present invention provides an RNAi agent for inhibiting LPAR1 gene expression, comprising a sense strand and an antisense strand that form a complementary duplex region, wherein the sense strand and antisense strand pair to form any one of the duplexes 8033, 8034, 8035, 8064, 8065, 8066, 8067, 8068, 8069, 8070, 8088, 8089, 8090, 8091, 8105, 8106, 8108, 8110, 8111, 8112, 8113, 8114, 8115, 8116, 8120, 8121, 8122, 8139, 8140, 8141, 8142, 8143, 8163, 8164, 8290, 8425, and 8426 as described herein.

### Nucleotide-Modified RNAi Agents

For any of the embodiments of the RNAi agents of the present invention described in the section "RNAi Agents For Inhibiting LPAR1 Gene Expression", the sense strand and/or antisense strand of the RNAi agent may comprise at least one modified nucleotide.

In some embodiments, the sense strand and the antisense strand of the RNAi agent for inhibiting LPAR1 gene expression provided by the present invention each comprise at least one modified nucleotide.

In some embodiments, each of the nucleotides in the sense strand and the antisense strand of the RNAi agent for inhibiting LPAR1 gene expression provided by the present invention is modified.

In any of the above embodiments, the modified nucleotide is independently selected from the group consisting of: 2'-deoxy-thymidine (dT) nucleotide, 2'-O-methyl-modified nucleotide (2'-OMe), 2'-fluoro-modified nucleotide (2'-F), 2'-deoxy-modified nucleotide, locked nucleic acid (LNA), unlocked nucleic acid (UNA), bridged nucleic acid (BNA), glycol nucleic acid (GNA), threose nucleic acid (TNA), conformationally restricted nucleotide, constrained ethyl nucleotide (cEt), 2'-amino-modified nucleotide, 2'-O-allyl-modified nucleotide, 2'-C-alkyl-modified nucleotide, 2'-O-methoxyethyl-modified nucleotide (2'-MOE), abasic nucleotide, inverted abasic nucleotide, inverted nucleotide, morpholino nucleotide (MOP), phosphoramidate (PN), tetrahydropyran-modified nucleotide (THP), 1,5-anhydrohexitol-modified nucleotide (HNA), cyclohexenyl-modified nucleotide, nucleotide comprising a phosphorothioate group (PS), nucleotide comprising a methylphosphonate group, nucleotide comprising a 5'-phosphate, nucleotide comprising a 5'-phosphate mimic, cationic lipid-conjugated nucleotide, nucleotide comprising a 5'-vinylphosphonate (5'-VP), and combinations thereof.

In preferred embodiments, the modified nucleotides are independently selected from the group consisting of 2'-O-methyl-modified nucleotides, 2'-fluoro-modified nucleotides, nucleotides comprising phosphorothioate internucleoside linkages, and combinations thereof. In preferred embodiments, every nucleotide of the sense strand and/or the antisense strand of the RNAi agent is modified. In preferred embodiments, every nucleotide of the sense strand and/or the antisense strand of the RNAi agent is modified, and the modified nucleotides are independently selected from the group consisting of 2'-O-methyl-modified nucleotides, 2'-fluoro-modified nucleotides, nucleotides comprising phosphorothioate internucleoside linkages, and combinations thereof.

In preferred embodiments, each of the nucleotides in the sense strand and the antisense strand of the RNAi agent for inhibiting LPAR1 gene expression provided by the present invention is modified, and the modification is selected from the group consisting of STC(Alnylam), ESC(Alnylam), Advanced ESC(Alnylam), ESC+(Alnylam), AD1-3(Arrowhead), AD5(Arrowhead) and GalXC(Dicerna) (see, e.g., Hu B, Zhong L, Weng Y, et al. Therapeutic siRNA: state of the art. Signal Transduct Target Ther. 2020; 5(1):101. Published 2020 Jun 19. doi:10.1038/s41392-020-0207-x).

In preferred embodiments, the 5'-terminal nucleotide of the antisense strand of the RNAi agent for inhibiting LPAR1 gene expression provided by the present invention contains a phosphate or phosphate analog modification, preferably 5'-VP (see Parmar R, Willoughby JL, Liu J, et al. 5'-(E)-Vinylphosphonate: A Stable Phosphate Mimic Can Improve the RNAi Activity of siRNA-GalNAc Conjugates. Chembiochem. 2016;17(11):985-989).

In some embodiments, the modified nucleobase may be a nucleobase having a cationic lipid covalently linked thereto. By introducing a modified nucleobase having a cationic lipid or an equivalent thereof covalently linked thereto, the antisense oligonucleotide can be rendered highly permeable to mammalian cell membranes. Suitable cationic lipid-linked modifications covalently attached thereto may be those described in WO2019022434A1, which is incorporated herein by reference in its entirety.

In some embodiments, in the RNAi agent for inhibiting LPAR1 gene expression provided by the present invention, each of the nucleotides of the antisense strand is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides.

In some embodiments, in the RNAi agent for inhibiting LPAR1 gene expression provided by the present invention, each of the nucleotides of the antisense strand is modified, and in the 5' to 3' direction, the nucleotides at positions 2, 5, 7, 12, and 14 of the antisense strand are 2'-fluoro-modified nucleotides, while the nucleotides at remaining positions of the antisense strand are all 2'-methoxy-modified nucleotides.

In some embodiments, in the RNAi agent for inhibiting LPAR1 gene expression provided by the present invention, each of the nucleotides of the antisense strand is modified, and in the 5' to 3' direction, the nucleotides at positions 2, 5, 7, 14, and 16 of the antisense strand are 2'-fluoro-modified nucleotides, while the nucleotides at remaining positions of the antisense strand are all 2'-methoxy-modified nucleotides.

In some embodiments, in the RNAi agent for inhibiting LPAR1 gene expression provided by the present invention, each of the nucleotides of the antisense strand is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides, and the antisense strand comprises at least one phosphorothioate internucleotide linkage. In preferable embodiments, the phosphorothioate internucleotide linkage is present at one or more of the following locations: (i) between nucleotide positions 1 and 2 at the 5'-end of the antisense strand; (ii) between nucleotide positions 2 and 3 at the 5'-end of the antisense strand; (iii) between nucleotide positions 1 and 2 at the 3'-end of the antisense strand; and (iv) between nucleotide positions 2 and 3 at the 3'-end of the antisense strand. In preferable embodiments, the phosphorothioate internucleotide linkage is present (i) between nucleotide positions 1 and 2 at the 5'-end of the antisense strand; (ii) between nucleotide positions 2 and 3 at the 5'-end of the antisense strand; (iii) between nucleotide positions 1 and 2 at the 3'-end of the antisense strand; and (iv) between nucleotide positions 2 and 3 at the 3'-end of the antisense strand.

In some embodiments, in the RNAi agent for inhibiting LPAR1 gene expression provided by the present invention, each of the nucleotides of the sense and antisense strands is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides; and in the 5' to 3' direction, the nucleotides at positions 7 and 9 of the sense strand are 2'-fluoro-modified nucleotides, and one or two of the nucleotides at positions 5, 8, and 11 of the sense strand is (are) 2'-fluoro-modified nucleotide(s), while the nucleotides at remaining positions of the sense strand are all 2'-methoxy-modified nucleotides.

For example, in the RNAi agent for inhibiting LPAR1 gene expression provided by the present invention, each of the nucleotides of the sense and antisense strands is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides; and in the 5' to 3' direction, the nucleotides at positions 5, 7, 8, and 9 of the sense strand are 2'-fluoro-modified nucleotides, while the nucleotides at remaining positions of the sense strand are all 2'-methoxy-modified nucleotides.

For example, in the RNAi agent for inhibiting LPAR1 gene expression provided by the present invention, each of the nucleotides of the sense and antisense strands is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides; and in the 5' to 3' direction, the nucleotides at positions 7, 8, and 9 of the sense strand are 2'-fluoro-modified nucleotides, while the nucleotides at remaining positions of the sense strand are all 2'-methoxy-modified nucleotides.

For example, in the RNAi agent for inhibiting LPAR1 gene expression provided by the present invention, each of the nucleotides of the sense and antisense strands is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides; and in the 5' to 3' direction, the nucleotides at positions 5, 7, and 9 of the sense strand are 2'-fluoro-modified nucleotides, while the nucleotides at remaining positions of the sense strand are all 2'-methoxy-modified nucleotides.

For example, in the RNAi agent for inhibiting LPAR1 gene expression provided by the present invention, each of the nucleotides of the sense and antisense strands is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides; and in the 5' to 3' direction, the nucleotides at positions 7, 9, and 11 of the sense strand are 2'-fluoro-modified nucleotides, while the nucleotides at remaining positions of the sense strand are all 2'-methoxy-modified nucleotides.

In preferred embodiments, the sense strand has at least one phosphorothioate internucleoside linkage. In preferred embodiments, the phosphorothioate internucleoside linkage is present in one or more of the following positions: (i) between nucleotide position 1 and nucleotide position 2 at the 5' end of the sense strand; (ii) between nucleotide position 2 and nucleotide position 3 at the 5' end of the sense strand; (iii) between nucleotide position 1 and nucleotide position 2 at the 3' end of the sense strand; and (iv) between nucleotide position 2 and nucleotide position 3 at the 3' end of the sense strand. In more preferred embodiments, the phosphorothioate internucleoside linkages are present between nucleotide position 1 and nucleotide position 2 at the 5' end of the sense strand; and between nucleotide position 2 and nucleotide position 3 at the 5' end of the sense strand. In more preferred embodiments, the phosphorothioate internucleoside linkages are present between nucleotide position 1 and nucleotide position 2 at the 5' end of the sense strand; between nucleotide position 2 and nucleotide position 3 at the 5' end of the sense strand; between nucleotide position 1 and nucleotide position 2 at the 3' end of the sense strand; and between nucleotide position 2 and nucleotide position 3 at the 3' end of the sense strand.

In preferred embodiments, the antisense strand has at least one phosphorothioate internucleoside linkage. Preferably, the phosphorothioate internucleoside linkage is present in one or more of the following: between nucleotide position 1 and nucleotide position 2 at the 5' end of the antisense strand; between nucleotide position 2 and nucleotide position 3 at the 5' end of the antisense strand; between nucleotide position 1 and nucleotide position 2 at the 3' end of the antisense strand; and between nucleotide position 2 and nucleotide position 3 at the 3' end of the antisense strand.

Accordingly, in some embodiments, in the RNAi agent for inhibiting LPAR1 gene expression provided by the present invention, each of the nucleotides of the sense and antisense strands is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides, and the antisense strand comprises at least one phosphorothioate internucleotide linkage, wherein the phosphorothioate internucleotide linkage is present at one or more of the following locations: between nucleotide positions 1 and 2 at the 5'-end of the antisense strand; between nucleotide positions 2 and 3 at the 5'-end of the antisense strand; between nucleotide positions 1 and 2 at the 3'-end of the antisense strand; and between nucleotide positions 2 and 3 at the 3'-end of the antisense strand; and in the 5' to 3' direction, the nucleotides at positions 7 and 9 of the sense strand are 2'-fluoro-modified nucleotides, and one or two of the nucleotides at positions 5, 8, and 11 of the sense strand is (are) 2'-fluoro-modified nucleotide(s), while the nucleotides at remaining positions of the sense strand are all 2'-methoxy-modified nucleotides; and the sense strand comprises at least one phosphorothioate internucleotide linkage, wherein the phosphorothioate internucleotide linkage is present between nucleotide positions 1 and 2 at the 5'-end of the sense strand and/or between nucleotide positions 2 and 3 at the 5'-end of the sense strand.

In some embodiments, in the RNAi agent for inhibiting LPAR1 gene expression provided by the present invention, each of the nucleotides of the sense and antisense strands is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides, and the antisense strand comprises at least one phosphorothioate internucleotide linkage, wherein the phosphorothioate internucleotide linkage is present: between nucleotide positions 1 and 2 at the 5'-end of the antisense strand; between nucleotide positions 2 and 3 at the 5'-end of the antisense strand; between nucleotide positions 1 and 2 at the 3'-end of the antisense strand; and between nucleotide positions 2 and 3 at the 3'-end of the antisense strand; and in the 5' to 3' direction, the nucleotides at positions 7 and 9 of the sense strand are 2'-fluoro-modified nucleotides, and one or two of the nucleotides at positions 5, 8, and 11 of the sense strand is (are) 2'-fluoro-modified nucleotide(s), while the nucleotides at remaining positions of the sense strand are all 2'-methoxy-modified nucleotides; and the sense strand comprises at least one phosphorothioate internucleotide linkage, wherein the phosphorothioate internucleotide linkage is present between nucleotide positions 1 and 2 at the 5'-end of the sense strand and between nucleotide positions 2 and 3 at the 5'-end of the sense strand.

### Ligand-Conjugated RNAi Agents

For any of the embodiments of the RNAi agent of the present invention described in the preceding sections entitled "RNAi Agents For Inhibiting LPAR1 Gene Expression" and "Nucleotide-modified RNAi agents", the RNAi agent may comprise a ligand. As used herein, a "ligand" refers to any compound or molecule capable of interacting, directly or indirectly, with another compound or molecule. The interaction of a ligand with another compound or molecule may elicit a biological response (e.g., initiation of a signal transduction cascade, induction of receptor-mediated endocytosis) or may simply be a physical attachment. A ligand may alter one or more properties of the attached double-stranded RNA molecule, such as the pharmacodynamics, pharmacokinetics, binding, absorption, cellular distribution, cellular uptake, charge, and/or clearance of the RNA molecule.

The LPAR1 gene is expressed in the various cells and tissues. Therefore, in certain embodiments, it is desirable to specifically deliver the RNAi agents of the present invention to hepatocytes. Accordingly, in certain embodiments, the ligand targets the specific delivery of the RNAi agent to hepatocytes using various methods described in more detail below. In certain embodiments, the RNAi agent is targeted to hepatocytes with a ligand that binds the surface-expressed asialoglycoprotein receptor (ASGR) or a component thereof (e.g., ASGR1, ASGR2).

In some embodiments, the RNAi agent can be specifically targeted to the liver by using a ligand that binds or interacts with a protein expressed on the surface of hepatocytes. For example, in certain embodiments, the ligand can comprise an antigen-binding protein (e.g., an antibody or binding fragment thereof such as Fab, scFv) that specifically binds a receptor expressed on hepatocytes, such as the asialoglycoprotein receptor and the LDL receptor. In one particular embodiment, the ligand comprises an antibody or binding fragment thereof that specifically binds ASGR1 and/or ASGR2. In another embodiment, the ligand comprises a Fab fragment of an antibody that specifically binds ASGR1 and/or ASGR2. In another embodiment, the ligand comprises a single-chain variable antibody fragment (scFv fragment) of an antibody that specifically binds ASGR1 and/or ASGR2. Exemplary antibodies that specifically bind ASGR1 and binding fragments thereof, which can serve as ligands for targeting the RNAi agents of the present invention to the liver, are described in WO 2017/058944, which is incorporated herein by reference in its entirety. Other antibodies or binding fragments thereof that specifically bind ASGR1, the LDL receptor, or other liver surface-expressed proteins suitable for use as ligands in the RNAi agents of the present invention are available from commercial sources.

In some embodiments, when used for extrahepatic delivery, including but not limited to, the central nervous system (CNS) (e.g., brain, spinal cord, or eye), muscle, lung, or adipose tissue, the RNAi agent may comprise one or more lipophilic groups conjugated to one or more positions of the antisense strand and/or sense strand. For example, the lipophilic group may be linked to the antisense strand and/or sense strand via a nucleobase, sugar moiety, or internucleoside linkage. In some embodiments, a lipophilic group having a phosphoramidite group is coupled to the 3' end or the 5' end of the sense strand or antisense strand in the final synthesis cycle. In some embodiments, the lipophilic group has an octanol-water partition coefficient exceeding 0, 1, 1.5, 2, 3, 4, 5, or 10. In some embodiments, the ligand is a lipophilic group, which is preferably selected from: lipids, vitamins, steroids, C₅-C₃₀ saturated or unsaturated fatty acids, C₅-C₃₀ alkyl groups, and polypeptides comprising at least one positively charged amino acid residue; said lipophilic group is more preferably selected from cholesterol, C₁₆ saturated or unsaturated fatty acids, C₁₆ alkyl groups, C₂₂ saturated or unsaturated fatty acids, or C₂₂ alkyl groups. Suitable lipophilic groups may be aliphatic, alicyclic, polyalicyclic compounds, steroids, linear or branched aliphatic hydrocarbon groups.

Exemplary lipophilic groups include, for example, lipophilic groups Y132 to Y135, Y158, Y165 to Y168, L10, L57, L321, L322, Q361 to Q367, Q361s to Q367s, Q370, Q377 to Q379, Q383, etc., as described in WO 2021/092371. Other ligands that may be conjugated to the RNAi agent of the present invention can be found in WO 2017/053995, WO 2019/217459, WO 2021/092371, WO 2017/053995, WO 2010/039548, WO 2023/064530, WO 2022/213118, and WO 2019/079386, each of which is incorporated herein by reference in its entirety.

In certain embodiments, the ligand comprises a carbohydrate. A "carbohydrate" refers to a compound composed of one or more monosaccharide units having at least 6 carbon atoms (which can be linear, branched, or cyclic) with oxygen, nitrogen, or sulfur atoms attached to each carbon atom. Carbohydrates include, but are not limited to, sugars (e.g., monosaccharides, disaccharides, trisaccharides, tetrasaccharides, and oligosaccharides containing about 4, 5, 6, 7, 8, or 9 monosaccharide units) and polysaccharides (such as starch, glycogen, cellulose, and polysaccharide gums). In some embodiments, the carbohydrate incorporated into the ligand is selected from pentoses, hexoses, or heptoses, and disaccharides and trisaccharides including such monosaccharide units. In other embodiments, the carbohydrate incorporated into the ligand is an amino sugar, such as galactosamine, glucosamine, N-acetylgalactosamine, and N-acetylglucosamine.

In some embodiments, the ligand comprises a hexose or a hexosamine. The hexose can be selected from glucose, galactose, mannose, fucose, or fructose. The hexosamine can be selected from fructosamine, galactosamine, glucosamine, or mannosamine. In certain embodiments, the ligand comprises glucose, galactose, galactosamine, or glucosamine. In one embodiment, the ligand comprises glucose, glucosamine, or N-acetylglucosamine. In another embodiment, the ligand comprises galactose, galactosamine, or N-acetylgalactosamine. In particular embodiments, ligands comprising glucose, galactose, and N-acetylgalactosamine (GalNAc) are particularly effective in targeting RNA to hepatocytes, as these ligands bind the ASGR expressed on the surface of hepatocytes. Examples of GalNAc- or galactose-containing ligands that can be incorporated into the RNAi agents of the present invention are described in U.S. Pat. Nos. 7,491,805, 8,106,022, and 8,877,917; U.S. Patent Application Publication No. US 20030130186; and WIPO Publication No. WO 2013/166155, all of which are incorporated herein by reference in their entirety.

In certain embodiments, the ligand comprises a multivalent carbohydrate moiety. As used herein, a "multivalent carbohydrate moiety" refers to a moiety comprising two or more carbohydrate units capable of independently binding or interacting with other molecules. For example, a multivalent carbohydrate moiety includes two or more binding domains composed of carbohydrates that can bind to two or more different molecules or two or more different sites on the same molecule. The "valency" of a carbohydrate moiety indicates the number of individual binding domains within the carbohydrate moiety. For example, the terms "monovalent," "bivalent," "trivalent," and "tetravalent" relative to a carbohydrate moiety refer to carbohydrate moieties having one, two, three, and four binding domains, respectively. A multivalent carbohydrate moiety can comprise a multivalent lactose moiety, a multivalent galactose moiety, a multivalent glucose moiety, a multivalent N-acetylgalactosamine moiety, a multivalent N-acetylglucosamine moiety, a multivalent mannose moiety, or a multivalent fucose moiety. In some embodiments, the ligand comprises a multivalent galactose moiety. In other embodiments, the ligand comprises a multivalent N-acetylgalactosamine moiety. In these and other embodiments, the multivalent carbohydrate moiety can be bivalent, trivalent, or tetravalent. In such embodiments, the multivalent carbohydrate moiety can be bi-branched or tri-branched. In one particular embodiment, the multivalent N-acetylgalactosamine moiety is trivalent or tetravalent. In another particular embodiment, the multivalent galactose moiety is trivalent or tetravalent. Exemplary trivalent and tetravalent GalNAc-containing ligands for incorporation into the RNAi agents of the present invention are described in detail below.

The ligand may also comprise an integrin ligand, for example, a ligand that specifically binds to an integrin, including but not limited to αVβ6. Suitable integrin ligands include, for example, those integrin ligands mentioned in WO2019089765A1 or WO2022056286A1, which are incorporated herein by reference in their entirety.

The ligand may also comprise a transferrin receptor 1 (TfR1) ligand, such as an anti-TfR1 antibody or polypeptide. Suitable TfR1 ligands include, for example, TfR1 antibodies described in WO2022147209A1 or WO2021154476A1, or bicyclic peptide ligands described in WO2022101633A1, and these patent documents are incorporated herein by reference in their entirety.

The ligand can be attached or conjugated to the RNA molecule of the RNAi agent directly or indirectly. For example, in some embodiments, the ligand is directly covalently attached to the the sense strand or the antisense strand of the RNAi agent. In other embodiments, the ligand is covalently attached to the the sense strand or the antisense strand of the RNAi agent via a linker. The ligand can be attached to a nucleobase, sugar moiety, or internucleotide linkage of the sense strand or antisense strand of the RNAi agent of the present invention.

In some embodiments, the ligand can be attached to 3' or 5'-end of the sense strand or antisense strand. In certain embodiments, the ligand is covalently attached to 5'-end of the sense strand. In such embodiments, the ligand is attached to 5'-terminal nucleotide of the sense strand. In these and other embodiments, the ligand is attached at 5'-position of 5'-terminal nucleotide of the sense strand. In other embodiments, the ligand is covalently attached to 3'- end of the sense strand. For example, in some embodiments, the ligand is attached to 3'-terminal nucleotide of the sense strand. In certain such embodiments, the ligand is attached at 3'-position of 3'-terminal nucleotide of the sense strand. In alternative embodiments, the ligand is attached near 3'-end but prior to one or more terminal nucleotides (i.e., one, two, three, or four nucleotides from the terminus). In some embodiments, the ligand is attached at the 2'-position of the sugar of 3'-terminal nucleotide of the sense strand. In other embodiments, the ligand is attached at the 2'-position of the sugar of 5'-terminal nucleotide of the sense strand.

In certain embodiments, the ligand is attached to the sense strand or antisense strand via a linker. A "linker" refers to an atom or group of atoms that covalently attaches the ligand to the polynucleotide component of the RNAi agent. The linker can have a length of about 1 to about 30 atoms, about 2 to about 28 atoms, about 3 to about 26 atoms, about 4 to about 24 atoms, about 6 to about 20 atoms, about 7 to about 20 atoms, about 8 to about 20 atoms, about 8 to about 18 atoms, and about 12 to about 18 atoms. In some embodiments, the linker can comprise a bifunctional linking moiety, typically comprising an alkyl group with two functional groups. One functional group is chosen to bind to the compound of interest (e.g., the sense strand or antisense strand of the RNAi agent), while the other functional group is chosen to bind to essentially any selected group, such as the ligands described herein. In certain embodiments, the linker comprises an oligomer of a chain structure or repeating units, such as ethylene glycol or amino acid units. Examples of functional groups commonly used in bifunctional linking moieties include, but are not limited to, electrophiles for reaction with nucleophilic groups and nucleophiles for reaction with electrophilic groups. In some embodiments, the bifunctional linking moiety includes an amino group, a hydroxyl group, a carboxylic acid, a thiol, an unsaturated bond (e.g., a double or triple bond), and the like.

Linkers suitable for attaching a ligand to the sense strand or antisense strand of the RNAi agent of the present invention include, but are not limited to, pyrrolidine, 8-amino-3,6-dioxaoctanoic acid, succinimidyl-4-(N-maleimidomethyl)cyclohexane-1-carboxylate, 6-aminohexanoic acid, substituted C₁-C₂₀ alkyl, substituted or unsubstituted C₂-C₂₀ alkenyl, or substituted or unsubstituted C₂-C₂₀ alkynyl. Preferred substituents for such linkers include, but are not limited to, hydroxyl, amino, alkoxy, carboxyl, benzyl, phenyl, nitro, thiol, thioalkoxy, halogen, alkyl, aryl, alkenyl, and alkynyl.

In certain embodiments, the linker is cleavable. A cleavable linker is one that is sufficiently stable outside the cell but is cleaved to release the two parts linked together by the linker upon entry into a target cell. In some embodiments, the cleavable linker is cleaved at least 10, 20, 30, 40, 50, 60, 70, 80, 90 times or more, or at least 100 times faster in the target cell or under a first reference condition (which can, for example, be selected to mimic or represent intracellular conditions) than in the blood of a subject.

Cleavable linkers are susceptible to cleavage agents, such as pH, redox potential, or the presence of degradative molecules. Generally, cleavage agents are more prevalent or found at higher levels or activity inside cells than in serum or blood. Examples of such cleavage agents include: redox agents, selected for specific substrates or lacking substrate specificity, including, for example, oxidases or reductases present in cells; esterases; endosomes or agents that can create an acidic environment, such as those resulting in a pH of 5 or lower; enzymes that can hydrolyze or degrade acid-cleavable linkers acting as general acids, peptidases (which can be substrate-specific), and phosphatases.

A cleavable linker can comprise a pH-sensitive moiety. Human serum has a pH of 7.4, while the average intracellular pH is slightly lower, ranging from about 7.1-7.3. Endosomes have a more acidic pH, in the range of 5.5-6.0, and lysosomes have an even more acidic pH (around 5.0). Some linkers will have a cleavable group that is cleaved at a preferred pH, thereby releasing the RNA molecule from the ligand inside the cell, or into a desired cellular organelle. The linker can include a cleavable group that is cleaved by a specific enzyme. The type of cleavable group incorporated into the linker can depend on the cell to be targeted. For example, a liver-targeting ligand can be linked to the RNA molecule via a linker comprising of an ester group. Hepatocytes are rich in esterases, so the linker is cleaved more efficiently in hepatocytes than in cell types not rich in esterases. Other cell types rich in esterases include cells of the lung, renal cortex, and testis. When targeting cells rich in peptidases, such as hepatocytes and synoviocytes, linkers containing peptide bonds can be used.

Other types of linkers suitable for attaching ligands to the sense strand or antisense strand in the RNAi agents of the present invention are known in the art, such as those described in U.S. Pat. Nos. 7,723,509, 8,017,762, 8,828,956, 8,877,917, and 9,181,551, all of which are incorporated herein by reference in their entirety.

In certain embodiments, the ligand covalently attached to the the sense strand or antisense strand of the RNAi agent of the present invention comprises a GalNAc moiety, such as a multivalent GalNAc. In some embodiments, the multivalent GalNAc moiety is a trivalent GalNAc and is attached to 3'-end of the sense strand. In other embodiments, the multivalent GalNAc moiety is a trivalent GalNAc and is attached to 5'-end of the sense strand. In other embodiments, the multivalent GalNAc moiety is a tetravalent GalNAc moiety and is attached to 3'-end of the sense strand. In other embodiments, the multivalent GalNAc moiety is a tetravalent GalNAc moiety and is attached to 5'-end of the sense strand.

In some embodiments, the ligand is L96, NAG25, and NAG37, having the formulas shown below, respectively, wherein the wave line represents a site to which the sense strand or antisense strand of the RNAi agent is attached.

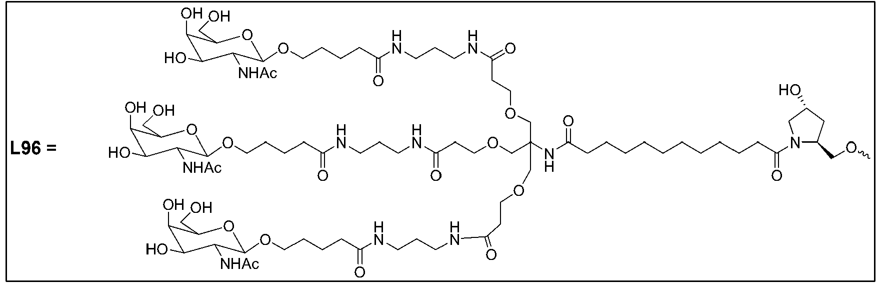

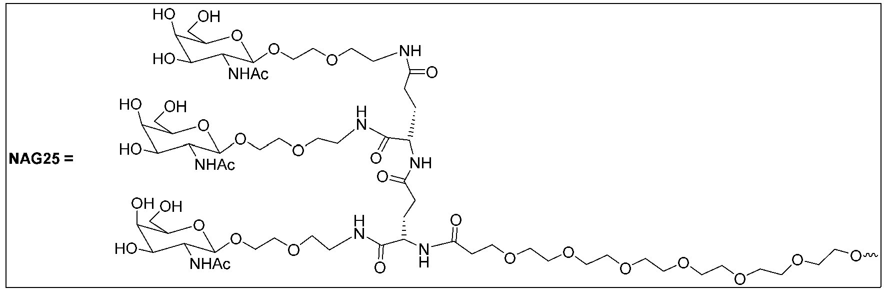

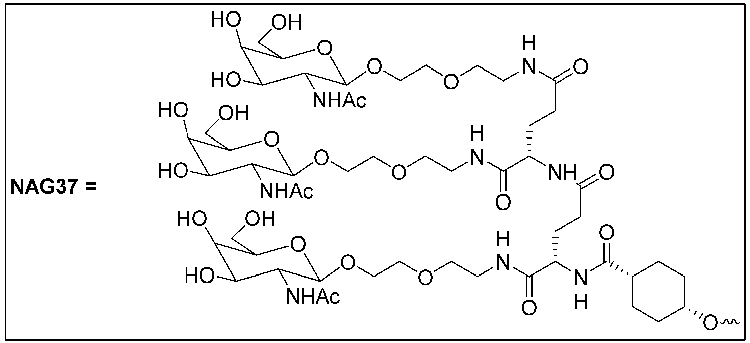

### Pharmaceutical Compositions

The present invention also encompasses pharmaceutical compositions and formulations comprising the RNAi agents described herein and a pharmaceutically acceptable carrier, excipient, or diluent. Such compositions and formulations can be used to reduce the expression of the LPAR1 gene in a patient in need thereof. Considering clinical application, the pharmaceutical compositions and formulations will be prepared in a form suitable for the intended application. Typically, this will involve preparing compositions substantially free of pyrogens and other impurities that may be harmful to humans or animals.

The ingredients and methods for formulating pharmaceutical compositions depend on many criteria, including, but not limited to, the route of administration, the type and extent of the disease or condition to be treated, or the dosage to be administered. In some embodiments, the pharmaceutical composition is formulated based on the intended delivery route. For example, in certain embodiments, the pharmaceutical composition is formulated for parenteral delivery. Parenteral administration forms include intravenous, intra-arterial, subcutaneous, intrathecal, intraperitoneal, or intramuscular injection or infusion. In one embodiment, the pharmaceutical composition is formulated for intravenous delivery. In such embodiments, the pharmaceutical composition may comprise a lipid-based delivery vehicle. In another embodiment, the pharmaceutical composition is formulated for subcutaneous delivery. In such embodiments, the pharmaceutical composition may comprise a targeting ligand (e.g., a GalNAc-containing or antibody-containing ligand as described herein).

In some embodiments, the pharmaceutical composition comprises an effective amount of the RNAi agent described herein. An "effective amount" refers to an amount sufficient to produce a beneficial or desired clinical outcome. In some embodiments, the effective amount is an amount sufficient to reduce the expression of the LPAR1 gene in a specific tissue or cell type of the patient (e.g., the liver or hepatocytes). The effective amount of the RNAi agent of the present invention can be from about 0.01 mg/kg body weight to about 100 mg/kg body weight, and can be administered daily, weekly, monthly, or at longer intervals. The precise determination of the specific effective dosage amount and administration frequency may be based on several factors, including the patient's body size, age, and general condition, the type of disease to be treated (e.g., myocardial infarction, coronary artery disease, peripheral artery disease, stroke), the specific RNAi agent used, and the route of administration.

Administration of the pharmaceutical compositions of the invention can be by any common route provided the target tissue is accessible via that route. These routes include, but are not limited to, parenteral (e.g., subcutaneous, intramuscular, intraperitoneal, or intravenous), oral, nasal, buccal, intradermal, transdermal, and sublingual routes, or by direct injection into liver tissue or delivery via the hepatic portal vein. In some embodiments, the pharmaceutical composition is administered parenterally. For example, in certain embodiments, the pharmaceutical composition is administered intravenously. In other embodiments, the pharmaceutical composition is administered subcutaneously.

Colloidal dispersion systems can be used as delivery vehicles for the RNAi agents of the present invention, such as macromolecular complexes, nanocapsules, microspheres, beads, and lipid-based systems, including oil-in-water emulsions, micelles, mixed micelles, and liposomes. Commercially available fat emulsions suitable for delivering the nucleic acids of the present invention include Intralipid (Baxter International Inc.), Liposyn (Abbott Pharmaceuticals), Lipsyn II (Hospira), Liposyn III (Hospira), Nutrilipid (B. Braun Medical Inc.), and other similar fat emulsions. A preferred colloidal system for use as an *in vivo* delivery vehicle is the liposome (i.e., an artificial membrane vesicle). The RNAi agents of the present invention can be encapsulated within liposomes or can form complexes therewith, particularly with cationic liposomes. Alternatively, the RNAi agents of the present invention can be complexed with lipids, particularly with cationic lipids. Suitable cationic lipids are, for example, dioleoyltrimethylammonium propane (DOTAP) and dioleoylphosphatidylethanolamine (DOTMA).

Liposomal formulations are particularly suitable for topical administration, with liposomes conferring several advantages over alternative formulations. Such advantages include reduced incidence of adverse effects associated with high systemic absorption of administered therapeutics, enhanced accumulation of administered therapeutics at desired target sites, and capability for dermal delivery of RNAi agents. In some embodiments, liposomes are employed to deliver RNAi agents to epidermal cells while concurrently enhancing permeation of said RNAi agents into dermal tissues, e.g., within the skin.

The RNAi agents of the present invention may be fully encapsulated within lipid-based formulations, such as LNPs or other nucleic acid-lipid particles. As used herein, the term "LNP" refers to stable nucleic acid-lipid particles. LNPs typically comprise a cationic lipid, a non-cationic lipid, and a lipid that prevents particle aggregation (e.g., a PEG-lipid conjugate). LNPs are particularly useful for systemic applications as they exhibit prolonged circulation times following intravenous (i.v.) injection and accumulate at distal sites (e.g., sites physically separated from the administration site). LNPs include "pSPLP," which comprise encapsulated condensing agent-nucleic acid complexes as described in WO00/03683. The LNP particles of the present invention generally possess a mean diameter ranging from about 50 nm to about 150 nm, more typically from about 60 nm to about 130 nm, more typically from about 70 nm to about 110 nm, and most typically from about 70 nm to about 90 nm, and are substantially non-toxic. Additionally, when nucleic acids are present within the nucleic acid-lipid particles of the invention, they demonstrate resistance to nuclease degradation in aqueous solutions. Nucleic acid-lipid particles and methods for their preparation are disclosed in, for example, U.S. Pat. Nos. 5,976,567; 5,981,501; 6,534,484; 6,586,410; 6,815,432; U.S. Publication No. 2010/0324120; and WO 96/40964. In one embodiment, the lipid-to-drug ratio (mass/mass basis) (e.g., lipid-to-RNAi agent ratio) will range from about 1:1 to about 50:1, about 1:1 to about 25:1, about 3:1 to about 15:1, about 4:1 to about 10:1, about 5:1 to about 9:1, or about 6:1 to about 9:1.

Cationic lipids may include, for example, N,N-dioleyl-N,N-dimethylammonium chloride (DODAC), N,N-distearyl-N,N-dimethylammonium bromide (DDAB), N-(1-(2,3-dioleyloxy)propyl)-N,N,N-trimethylammonium chloride (DOTAP), N-(1-(2,3-dioleyloxy)propyl)-N,N,N-trimethylammonium chloride (DOTMA), N,N-dimethyl-2,3-dioleyloxy)propylamine (DODMA), 1,2-dioleyloxy-N,N-dimethylaminopropane (DLinDMA), 1,2-dilinoleyloxy-N,N-dimethylaminopropane (DLenDMA), 1,2-dilinoleylcarbamoyloxy-3-dimethylaminopropane (DLin-C-DAP), 1,2-dilinoleyloxy-3-(dimethylamino)acetoxypropane (DLin-DAC), 1,2-dilinoleyloxy-3-morpholinopropane (DLin-MA), 1,2-dilinolenyl-3-dimethylaminopropane (DLinDAP), 1,2-dilinoleylthio-3-dimethylaminopropane (DLin-S-DMA), 1-linoleoyl-2-linoleyloxy-3-dimethylaminopropane (DLin-2-DMAP), 1,2-dilinoleyloxy-3-trimethylaminopropane chloride salt (DLin-TMA.Cl), 1,2-dilinoleoyl-3-trimethylaminopropane chloride salt (DLin-TAP.Cl), 1,2-dilinoleoyloxy-3-(N-methylpiperazinyl)propane (DLin-MPZ), 3-(N,N-dilinoleylamino)-1,2-propanediol (DLinAP), 3-(N,N-dioleylamino)-1,2-propanediol (DOAP), 1,2-dilinoleyloxo-3-(2-N,N-dimethylamino)ethoxypropane (DLin-EG-DMA), 1,2-dilinolenyloxy-N,N-dimethylaminopropane (DLinDMA), 2,2-dilinoleyl-4-dimethylaminomethyl-[1,3]-dioxolane (DLin-K-DMA) or analogs thereof, (3aR,5s,6aS)-N,N-dimethyl-2,2-di((9Z,12Z)-octadeca-9,12-dienyl)tetrahydro-3aH-cyclopenta[d][1,3]dioxol-5-amine (ALN100), (6Z,9Z,28Z,31Z)-heptatriaconta-6,9,28,31-tetraen-19-yl 4-(dimethylamino)butanoate (MC3), 1,1'-(2-(4-(2-((2-(bis(2-(bis(2-hydroxydodecyl)amino)ethyl)(2-hydroxydodecyl)amino)ethyl)piperazin-1-yl)ethylazanediyl)didodecan-2-ol (Tech G1), or mixtures thereof. The cationic lipid may constitute about 20 mol% to about 50 mol%, or about 40 mol% of the total lipid present in the particle.

In some embodiments, the compound 2,2-dilinoleyl-4-dimethylaminomethyl-[1,3]-dioxolane may be utilized in the preparation of lipid-siRNA nanoparticles. In certain embodiments, the lipid-siRNA particles comprise 40% 2,2-dilinoleyl-4-dimethylaminomethyl-[1,3]-dioxolane : 10% DSPC : 40% cholesterol : 10% PEG-C-DOMG (mol%), with a particle size of 63.0 ± 20 nm and an siRNA-to-lipid ratio of 0.027.

Ionizable/non-cationic lipids may be anionic lipids or neutral lipids, including but not limited to distearoylphosphatidylcholine (DSPC), dioleoylphosphatidylcholine (DOPC), dipalmitoylphosphatidylcholine (DPPC), dioleoylphosphatidylglycerol (DOPG), dipalmitoyl (DP), dioleoylphosphatidylethanolamine (DOPE), palmitoyloleoylphosphatidylcholine (POPC), palmitoyloleoylphosphatidylethanolamine (POPE), N-(4-(p-maleimidophenyl)butyryl)-dioleoylphosphatidylethanolamine (DOPE-mal), dipalmitoylphosphatidylethanolamine (DPPE), dimyristoylphosphatidylethanolamine (DMPE), distearoylphosphatidylethanolamine (DSPE), 16-O-monomethyl PE, 16-O-dimethyl PE, 18-1-trans PE, 1-stearoyl-2-oleoyl-phosphatidylethanolamine (SOPE), cholesterol, or mixtures thereof. The non-cationic lipid may constitute about 5 mol% to about 90 mol%, about 10 mol%, or about 58 mol% (when cholesterol is included) of the total lipid present in the particle.

Lipids conjugated to prevent particle aggregation may include, for example, polyethylene glycol (PEG)-lipids, comprising but not limited to PEG-diacylglycerol (PEG-DAG), PEG-dialkoxypropyl (PEG-DAA), PEG-phospholipid, PEG-ceramide (PEG-Cer), or mixtures thereof. PEG-DAA conjugates may include, for instance, PEG-didodecyloxypropyl (C12), PEG-dimyristyloxypropyl (C14), PEG-dipalmityloxypropyl (C14), or PEG-distearoyloxypropyl (C18). The lipid conjugated to prevent particle aggregation may constitute 0 mol% to about 20 mol%, or about 2 mol% of the total lipid present in the particle. In some embodiments, the nucleic acid-lipid particles further comprise cholesterol, for example constituting about 10 mol% to about 60 mol%, or about 48 mol% of the total lipid present in said particle.

In one embodiment, the lipidoid ND98·4HCl (molecular weight 1487) (see U.S. Patent Application No. 12/056,230, incorporated herein by reference in its entirety), cholesterol (Sigma-Aldrich), and PEG-ceramide C16 (Avanti Polar Lipids) may be utilized to prepare lipid-dsRNA nanoparticles (i.e., LNP01 particles). Each stock solution in ethanol may be prepared as follows: ND98 at 133 mg/mL; cholesterol at 25 mg/mL; PEG-ceramide C16 at 100 mg/mL. The stock solutions of ND98, cholesterol, and PEG-ceramide C16 may then be mixed at a mol ratio of, for example, 42:48:10. The combined lipid solution may be mixed with aqueous siRNA (e.g., in sodium acetate buffer at pH 5) such that the final ethanol concentration is about 35-45% and the final sodium acetate concentration is about 100-300 mM. Lipid-siRNA nanoparticles typically form spontaneously upon mixing.

Other exemplary lipid-siRNA formulations are available from *e.g.* WO2009/127060(SNALP), PCT/US2010/022614(XTC), US 2010/0324120(MC3), PCT/US09/63933(ALNY-100) and WO2010/129709(C12-200).

In some embodiments, the RNAi agent of the present invention may be conjugated to a cell-penetrating peptide (CPP), wherein said CPP may comprise at least one positively charged amino acid or ionizable amino acid, such as arginine, to form a peptide-oligonucleotide conjugate. Suitable CPPs for forming peptide-oligonucleotide conjugates may be found, for example, in those described in WO2022213118A1 or WO2019079386A1, which are incorporated herein by reference in their entirety.

Pharmaceutical compositions suitable for injectable use comprise, for example, sterile aqueous solutions or dispersions and sterile powders for the extemporaneous preparation of sterile injectable solutions or dispersions. Generally, such formulations are sterile and possess sufficient fluidity for ease of syringeability. The formulations shall remain stable under manufacturing and storage conditions and shall be preserved against the contaminating action of microorganisms such as bacteria and fungi. Suitable solvents or dispersion media may include, for instance, water, ethanol, polyhydric alcohols (e.g., glycerol, propylene glycol, liquid polyethylene glycols, and the like), suitable mixtures thereof, and vegetable oils. Appropriate fluidity may be maintained, for example, using a coating material such as lecithin, by maintaining required particle size in dispersions, or by employing surfactants. Prevention of microbial action may be achieved through various antibacterial and antifungal agents, including but not limited to parabens, chlorobutanol, phenol, sorbic acid, thimerosal, and the like. In many cases, it is preferable to include isotonic agents, such as sugars or sodium chloride. Prolonged absorption of injectable compositions may be accomplished by incorporating agents delaying absorption in the composition, for example, aluminum monostearate and gelatin.

Sterile injectable solutions may be prepared by incorporating the requisite quantity of the active compound with any additional ingredients (e.g., those enumerated supra) into an appropriate solvent, followed by filtration sterilization. Generally, dispersions are prepared by incorporating various sterilized active ingredients into a sterile vehicle medium containing essential dispersion agents and other requisite components, for instance, as described above. In the case of sterile powders for preparing sterile injectable solutions, preferred preparation methods include vacuum desiccation and lyophilization techniques, which yield powders of the active ingredient(s) together with any additional desired constituents from their prior sterile-filtered solutions.

The compositions of the present invention may generally be formulated in neutral form or as pharmaceutically acceptable salts. Such salts include, for example, acid addition salts (formed with free amino groups) derived from inorganic acids (e.g., hydrochloric or phosphoric acids) or organic acids (e.g., acetic, oxalic, tartaric, mandelic acids, etc.). Salts formed with free carboxyl groups may likewise be derived from inorganic bases (e.g., sodium, potassium, ammonium, calcium, or ferric hydroxides) or organic bases (e.g., isopropylamine, trimethylamine, histidine, procaine, etc.). In some embodiments, the RNAi agent of the invention is formulated as a sodium salt.

For parenteral administration in aqueous solution form, for example, the solution is typically appropriately buffered, and the liquid diluent is first rendered isotonic with sufficient saline or glucose, for instance. Such aqueous solutions may be employed for intravenous, intramuscular, subcutaneous, and intraperitoneal administration, among others. Preferably, sterile aqueous media are utilized. By way of example, a unit dose may be dissolved in 1 mL of isotonic NaCl solution and either added to 1000 mL of subcutaneous infusion fluid or injected at the proposed infusion site. For administration to humans, the formulations shall comply with sterility, pyrogenicity, general safety, and purity standards as required by local Food and Drug Administration regulations. In certain embodiments, the pharmaceutical composition of the invention comprises the sterile saline solution described herein and the RNAi agent, or consists essentially of these components. In other embodiments, the pharmaceutical composition of the invention comprises the RNAi agent described herein and sterile water (e.g., Water for Injection, WFI), or consists essentially thereof. In yet other embodiments, the pharmaceutical composition of the invention comprises the RNAi agent described herein and Phosphate-Buffered Saline (PBS), or consists essentially thereof.

In certain embodiments, the pharmaceutical compositions of the present invention are packaged with or stored within an administration device. Devices for injectable formulations comprise, but are not limited to, injection ports, pre-filled syringes, auto-injectors, infusion pumps, implantable injectors, and injection pens. Devices for nebulized or powdered formulations comprise, but are not limited to, inhalers, insufflators, nebulizers, and the like. Accordingly, the present invention encompasses an administration device containing the pharmaceutical composition of the invention, for treating or preventing one or more diseases or disorders as described herein.

### Therapeutic Methods and Uses

The present invention provides a method for reducing or inhibiting the expression of LPAR1 gene in a cell (e.g., a hepatocyte) by contacting the cell with any of the RNAi agents described herein. The cell can be *in vitro* or *in vivo*. LPAR1 gene expression can be assessed by measuring the amount or level of LPAR1 mRNA or LPAR1 protein. The reduction in LPAR1 expression in cells or animals treated with the RNAi agents of the invention can be determined relative to LPAR1 expression in cells and animals not treated with the RNAi agent or treated with a control RNAi agent. For example, in some embodiments, the reduction in LPAR1 expression is assessed by (a) measuring the amount or level of LPAR1 mRNA in hepatocytes treated with an RNAi agent of the invention, (b) measuring the amount or level of LPAR1 mRNA in hepatocytes treated with a control RNAi agent (e.g., an RNAi agent targeting an RNA molecule not expressed in hepatocytes or an RNAi agent having a nonsense or scrambled sequence) or no RNAi agent, and (c) comparing the LPAR1 mRNA level measured in the treated cells of (a) with the LPAR1 mRNA level in the control cells of (b). The LPAR1 mRNA levels in the treated and control cells may be normalized to the RNA level of a control gene (e.g., 18S ribosomal RNA or a housekeeping gene) prior to comparison. LPAR1 mRNA levels can be measured by various methods, including Northern blot analysis, nuclease protection assay, fluorescent in situ hybridization (FISH), reverse transcriptase (RT)-PCR, real-time RT-PCR, quantitative PCR, droplet digital PCR, and the like.

In some embodiments, the method for assessing LPAR1 expression levels is performed *in vitro* in cells that naturally express the LPAR1 gene (e.g., hepatocytes) or cells that have been engineered to express LPAR1.

In other embodiments, the method for assessing LPAR1 expression levels is performed *in vivo*. The RNAi agent and any control RNAi agent can be administered to an animal (e.g., a transgenic animal expressing the LPAR1 gene or a non-human primate), and LPAR1 mRNA or serum LPAR1 protein levels are assessed in liver tissue harvested from the animal posttreatment. Alternatively, or additionally, biomarkers or functional phenotypes associated with LPAR1 expression can be assessed in the treated animal. For example, LPAR1 protein is the expression product of LPAR1 mRNA in serum or plasma. Therefore, the serum or plasma levels of LPAR1 protein can be measured in an animal treated with an RNAi agent of the invention to assess the functional efficacy of reducing LPAR1 expression.

In certain embodiments, the expression of LPAR1 in hepatocytes is reduced by the RNAi agent of the invention by at least 40%, at least 45%, or at least 50%. In some embodiments, the expression of LPAR1 in hepatocytes is reduced by the RNAi agent of the invention by at least 60%, at least 65%, at least 70%, at least 75%, at least 80%, or at least 85%. In other embodiments, the expression of LPAR1 in hepatocytes is reduced by the RNAi agent of the invention by about 90% or more, e.g., 91%, 92%, 93%, 94%, 95%, 96%, 97%, 98%, 99%, or more. The percentage reduction in LPAR1 expression can be measured by any of the methods described herein, as well as other methods known in the art.

The present invention provides methods for reducing or inhibiting the expression of the LPAR1 gene, thereby reducing or inhibiting the production of LPAR1 protein, in a patient in need thereof, as well as methods for treating or preventing a disease or disorder associated with LPAR1 expression or activity. A "disease or disorder associated with LPAR1 expression" refers to a disease or disorder characterized by altered levels of LPAR1 expression, or a condition where elevated levels of LPAR1 expression are associated with an increased risk of developing the disease or disorder. In certain embodiments, the RNAi agents of the invention are particularly useful for treating or preventing metabolic diseases and reducing circulating levels of LPAR1.

Diseases and disorders associated with LPAR1 expression that can be treated or prevented according to the methods of the invention include, but are not limited to, fibrotic diseases (e.g., pulmonary fibrosis, hepatic fibrosis, renal fibrosis), hypertrophic cardiomyopathy, diffuse scleroderma, adult-onset scleroderma, cancers (e.g., hepatocellular carcinoma, ovarian cancer, glioblastoma), diabetic nephropathy, atherosclerosis, rheumatoid arthritis, neuropathic pain, non-alcoholic steatohepatitis, benign prostatic hyperplasia, and demyelination.

In certain embodiments, the present invention provides a method for reducing LPAR1 expression in a patient in need thereof, comprising administering to the patient any RNAi agent described herein. Preferably, the level of LPAR1 expression in the patient's hepatocytes is reduced following administration of the RNAi agent, compared to the level of LPAR1 expression in a patient not receiving the RNAi agent, or compared to the level of LPAR1 expression in the patient prior to administration of the RNAi agent. In some embodiments, after administration of the RNAi agent of the invention, LPAR1 expression in the patient is reduced by at least 30%, at least 35%, at least 40%, at least 45%, at least 50%, at least 55%, at least 60%, at least 65%, at least 70%, at least 75%, at least 80%, at least 85%, or at least 90%, e.g., 91%, 92%, 93%, 94%, 95%, 96%, 97%, 98%, or 99%. The percentage reduction in LPAR1 expression can be measured by any of the methods described herein, as well as other methods known in the art. In certain embodiments, the percentage reduction in LPAR1 expression is determined by assessing the level of LPAR1 protein in the patient's serum or plasma according to the methods described herein.

In certain embodiments, a patient in need of reducing LPAR1 expression is a patient diagnosed with or at risk of developing a disease mediated by aberrant expression levels of LPAR1. Accordingly, the present invention includes a method of treating or preventing a disease mediated by aberrant expression levels of LPAR1 in a patient in need thereof by administering any RNAi agent of the present invention. In some embodiments, the present invention includes use of any RNAi agent described herein in the manufacture of a medicament for treating or preventing a disease mediated by aberrant expression levels of LPAR1 in a patient in need thereof. In other embodiments, the present invention provides an LPAR1-targeting RNAi agent for use in a method of treating or preventing a disease mediated by aberrant expression levels of LPAR1 in a patient in need thereof. As described above, the disease mediated by aberrant expression levels of LPAR1 is, for example, fibrotic diseases (e.g., pulmonary fibrosis, hepatic fibrosis, renal fibrosis), hypertrophic cardiomyopathy, diffuse scleroderma, adult-onset scleroderma, cancers (e.g., hepatocellular carcinoma, ovarian cancer, glioblastoma), diabetic nephropathy, atherosclerosis, rheumatoid arthritis, neuropathic pain, non-alcoholic steatohepatitis, benign prostatic hyperplasia, and demyelination.

In certain other embodiments, a patient in need of reducing LPAR1 expression is a patient with elevated levels of LPAR1. Accordingly, in some embodiments, the present invention provides a method of reducing LPAR1 protein levels in a patient in need thereof by administering to the patient any RNAi agent described herein. In some embodiments, the present invention includes use of any RNAi agent described herein in the manufacture of a medicament for reducing LPAR1 protein levels in a patient in need thereof. In other embodiments, the present invention provides an LPAR1-targeting RNAi agent for use in a method of reducing LPAR1 protein levels in a patient in need thereof.

In some embodiments of the present invention, the present invention provides use of an agent that reduces the expression of the gene encoding LPAR1 in the manufacture of a medicament for treating a disease or disorder treatable by LPAR1 knockdown. In preferred embodiments, said agent is an RNAi agent. In preferred embodiments, said disease or disorder is selected from the group consisting of fibrotic diseases (e.g., pulmonary fibrosis, hepatic fibrosis, renal fibrosis), hypertrophic cardiomyopathy, diffuse scleroderma, adult-onset scleroderma, cancers (e.g., hepatocellular carcinoma, ovarian cancer, glioblastoma), diabetic nephropathy, atherosclerosis, rheumatoid arthritis, neuropathic pain, non-alcoholic steatohepatitis, benign prostatic hyperplasia, and demyelination.

Therefore, in some embodiments, the present invention provides use of an RNAi agent that reduces the expression of the gene encoding LPAR1 in the manufacture of a medicament for treating a disease or disorder selected from the group consisting of fibrotic diseases (e.g., pulmonary fibrosis, hepatic fibrosis, renal fibrosis), hypertrophic cardiomyopathy, diffuse scleroderma, adult-onset scleroderma, cancers (e.g., hepatocellular carcinoma, ovarian cancer, glioblastoma), diabetic nephropathy, atherosclerosis, rheumatoid arthritis, neuropathic pain, non-alcoholic steatohepatitis, benign prostatic hyperplasia, and demyelination.

### Sequence Listings

**Table 1. Nucleotide Unmodified LPAR1 siRNA**

| **Duplex** | **Binding Positions** | **Sense Strand** | **SS 5'-3'** | **SEQ ID NO** | **Antisense Strand** | **AS 5'-3'** | **SEQ ID NO** |
|---|---|---|---|---|---|---|---|
| 8033 | 605-625 | 8033-S0 | CCAGUUCACAG CCAUGAAU | 3 | 8033-A0 | AUUCAUGGCUGUGA ACUGGGG | 40 |
| 8034 | 607-627 | 8034-S0 | AGUUCACAGCC AUGAAUGA | 4 | 8034-A0 | UCAUUCAUGGCUGU GAACUGG | 41 |
| 8035 | 608-628 | 8035-S0 | GUUCACAGCCA UGAAUGAA | 5 | 8035-A0 | UUCAUUCAUGGCUG UGAACUG | 42 |
| 8064 | 796-816 | 8064-S0 | GCCGCUUCCAU UUUCCUAU | 6 | 8064-A0 | AUAGGAAAAUGGAA GCGGCGG | 43 |
| 8065 | 797-817 | 8065-S0 | CCGCUUCCAUU UUCCUAUU | 7 | 8065-A0 | AAUAGGAAAAUGGA AGCGGCG | 44 |
| 8066 | 798-818 | 8066-S0 | CGCUUCCAUUU UCCUAUUU | 8 | 8066-A0 | AAAUAGGAAAAUGG AAGCGGC | 45 |
| 8067 | 799-819 | 8067-S0 | GCUUCCAUUUU CCUAUUUA | 9 | 8067-A0 | UAAAUAGGAAAAUG GAAGCGG | 46 |
| 8068 | 800-820 | 8068-S0 | CUUCCAUUUUC CUAUUUAU | 10 | 8068-A0 | AUAAAUAGGAAAAU GGAAGCG | 47 |
| 8069 | 802-822 | 8069-S0 | UCCAUUUUCCU AUUUAUUA | 11 | 8069-A0 | UAAUAAAUAGGAAA AUGGAAG | 48 |
| 8070 | 836-856 | 8070-S0 | GGCUGCUGCAG ACUUCUUU | 12 | 8070-A0 | AAAGAAGUCUGCAG CAGCCAG | 49 |
| 8088 | 1110-1130 | 8088-S0 | AGUGUGGGCUG GAACUGUA | 13 | 8088-A0 | UACAGUUCCAGCCC ACACUGG | 50 |
| 8089 | 1111-1131 | 8089-S0 | GUGUGGGCUGG AACUGUAU | 14 | 8089-A0 | AUACAGUUCCAGCC CACACUG | 51 |
| 8090 | 1119-1139 | 8090-S0 | UGGAACUGUAU CUGUGAUA | 15 | 8090-A0 | UAUCACAGAUACAG UUCCAGC | 52 |
| 8091 | 1120-1140 | 8091-S0 | GGAACUGUAUC UGUGAUAU | 16 | 8091-A0 | AUAUCACAGAUACA GUUCCAG | 53 |
| 8105 | 1170-1190 | 8105-S0 | AGUGACUCUUA CUUAGUCU | 17 | 8105-A0 | AGACUAAGUAAGAG UCACUGU | 54 |
| 8106 | 1171-1191 | 8106-S0 | GUGACUCUUAC UUAGUCUU | 18 | 8106-A0 | AAGACUAAGUAAGA GUCACUG | 55 |
| 8108 | 1214-1234 | 8108-S0 | CUUUGUGGUAA UGGUGGUU | 19 | 8108-A0 | AACCACCAUUACCA CAAAGGU | 56 |
| 8110 | 1228-1248 | 8110-S0 | UGGUUCUCUAU GCUCACAU | 20 | 8110-A0 | AUGUGAGCAUAGAG AACCACC | 57 |
| 8111 | 1232-1252 | 8111-S0 | UCUCUAUGCUC ACAUCUUU | 21 | 8111-A0 | AAAGAUGUGAGCAU AGAGAAC | 58 |
| 8112 | 1241-1261 | 8112-S0 | UCACAUCUUUG GCUAUGUU | 22 | 8112-A0 | AACAUAGCCAAAGA UGUGAGC | 59 |
| 8113 | 1255-1275 | 8113-S0 | AUGUUCGCCAG AGGACUAU | 23 | 8113-A0 | AUAGUCCUCUGGCG AACAUAG | 60 |
| 8114 | 1261-1281 | 8114-S0 | GCCAGAGGACU AUGAGAAU | 24 | 8114-A0 | AUUCUCAUAGUCCU CUGGCGA | 61 |
| 8115 | 1263-1283 | 8115-S0 | CAGAGGACUAU GAGAAUGU | 25 | 8115-A0 | ACAUUCUCAUAGUC CUCUGGC | 62 |
| 8116 | 1265-1285 | 8116-S0 | GAGGACUAUGA GAAUGUCU | 26 | 8116-A0 | AGACAUUCUCAUAG UCCUCUG | 63 |
| 8120 | 1329-1349 | 8120-S0 | CUUCUGAAGAC UGUGGUCA | 27 | 8120-A0 | UGACCACAGUCUUC AGAAGAC | 64 |
| 8121 | 1331-1351 | 8121-S0 | UCUGAAGACUG UGGUCAUU | 28 | 8121-A0 | AAUGACCACAGUCU UCAGAAG | 65 |
| 8122 | 1337-1357 | 8122-S0 | GACUGUGGUCA UUGUGCUU | 29 | 8122-A0 | AAGCACAAUGACCA CAGUCUU | 66 |
| 8139 | 1492-1512 | 8139-S0 | UUUACUCCUAC CGCGACAA | 30 | 8139-A0 | UUGUCGCGGUAGGA GUAAAUG | 67 |
| 8140 | 1496-1516 | 8140-S0 | CUCCUACCGCG ACAAAGAA | 31 | 8140-A0 | UUCUUUGUCGCGGU AGGAGUA | 68 |
| 8141 | 1497-1517 | 8141-S0 | UCCUACCGCGA CAAAGAAA | 32 | 8141-A0 | UUUCUUUGUCGCGG UAGGAGU | 69 |
| 8142 | 1498-1518 | 8142-S0 | CCUACCGCGAC AAAGAAAU | 33 | 8142-A0 | AUUUCUUUGUCGCG GUAGGAG | 70 |
| 8143 | 1500-1520 | 8143-S0 | UACCGCGACAA AGAAAUGA | 34 | 8143-A0 | UCAUUUCUUUGUCG CGGUAGG | 71 |
| 8163 | 1780-1800 | 8163-S0 | ACUAACCAAUG ACAGUAUU | 35 | 8163-A0 | AAUACUGUCAUUGG UUAGUGU | 72 |
| 8164 | 1781-1801 | 8164-S0 | CUAACCAAUGA CAGUAUUU | 36 | 8164-A0 | AAAUACUGUCAUUG GUUAGUG | 73 |
| 8290 | 2267-2287 | 8290-S0 | AAUCUUCUAGG AAAUAGAA | 37 | 8290-A0 | UUCUAUUUCCUAGA AGAUUUA | 74 |
| 8425 | 2789-2809 | 8425-S0 | CUAUAUUAUAU UCCAUUUA | 38 | 8425-A0 | UAAAUGGAAUAUAA UAUAGCC | 75 |
| 8426 | 2790-2810 | 8426-S0 | UAUAUUAUAUU CCAUUUAA | 39 | 8426-A0 | UUAAAUGGAAUAUA AUAUAGC | 76 |

### Human LPAR1, mRNA (SEQ ID NO. 1, NCBI Accession No. NM_00135 1411.2)

1 agtaggggcc gactggcggc tgacgctccc tgagtggcga ctccgtctcc agccccgctg
61 cggagcgcgg gccggatctg gggcggccag ggcccggagc cgcggagccc tccccgccgc
121 ccggccgagc acgggacccc ggcggggtgg gcgcaggggg cggccccgct ctgggcgact

181 gccgaggggc gggcggaggg ccgggctcgg ctggcggtgg ggcgggggcc gccgggactg
241 ggcgcgcggc ctgaagccag cccgggggcg gcaggagagg gacgcgcggc ggcagcgagc
301 gcagggaaca gctcctgccc aggtctgtgg gcactcagca tggatttcag tctccctgtg
361 agtgatggga aagaactaac agaggtggac gtctgattta tgaagctccc catccaccta
421 tctgagtacc tgacttctca ggactgacac ctacagcatc aggtacacag cttctcctag
481 catgacttcg atctgatcag caaacaagaa aatttgtctc ccgtagttct ggggcgtgtt
541 caccacctac aaccacagag ctgtcatggc tgccatctct acttccatcc ctgtaatttc
601 acagccccag ttcacagcca tgaatgaacc acagtgcttc tacaacgagt ccattgcctt
661 cttttataac cgaagtggaa agcatcttgc cacagaatgg aacacagtca gcaagctggt
721 gatgggactt ggaatcactg tttgtatctt catcatgttg gccaacctat tggtcatggt
781 ggcaatctat gtcaaccgcc gcttccattt tcctatttat tacctaatgg ctaatctggc
841 tgctgcagac ttctttgctg ggttggccta cttctatctc atgttcaaca caggacccaa
901 tactcggaga ctgactgtta gcacatggct ccttcgtcag ggcctcattg acaccagcct
961 gacggcatct gtggccaact tactggctat tgcaatcgag aggcacatta cggttttccg
1021 catgcagctc cacacacgga tgagcaaccg gcgggtagtg gtggtcattg tggtcatctg
1081 gactatggcc atcgttatgg gtgctatacc cagtgtgggc tggaactgta tctgtgatat
1141 tgaaaattgt tccaacatgg cacccctcta cagtgactct tacttagtct tctgggccat
1201 tttcaacttg gtgacctttg tggtaatggt ggttctctat gctcacatct ttggctatgt
1261 tcgccagagg actatgagaa tgtctcggca tagttctgga ccccggcgga atcgggatac
1321 catgatgagt cttctgaaga ctgtggtcat tgtgcttggg gcctttatca tctgctggac
1381 tcctggattg gttttgttac ttctagacgt gtgctgtcca cagtgcgacg tgctggccta
1441 tgagaaattc ttccttctcc ttgctgaatt caactctgcc atgaacccca tcatttactc
1501 ctaccgcgac aaagaaatga gcgccacctt taggcagatc ctctgctgcc agcgcagtga
1561 gaaccccacc ggccccacag aaggctcaga ccgctcggct tcctccctca accacaccat
1621 cttggctgga gttcacagca atgaccactc tgtggtttag aacggaaact gagatgagga
1681 accagccgtc ctctcttgga ggataaacag cctcccccta cccaattgcc agggcaaggt
1741 ggggtgtgag agaggagaaa agtcaactca tgtacttaaa cactaaccaa tgacagtatt
1801 tgttcctgga ccccacaaga cttgatatat attgaaaatt agcttatgtg acaaccctca
1861 tcttgatccc catcccttct gaaagtagga agttggagct cttgcaatgg aattcaagaa
1921 cagactctgg agtgtccatt tagactacac taactagact tttaaaagat tttgtgtggt
1981 ttggtgcaag tcagaataaa ttctggctag ttgaatccac aacttcattt atatacaggc
2041 ttcccttttt tatttttaaa ggatacgttt cacttaataa acacgtttat gcctatcagc
2101 atgtttgtga tggatgagac tatggactgc ttttaaacta ccataattcc atttttccc
2161 ttacatagga aaactgtaag ttggaattat cttttgttta gaaagcatgc atgtaatgta
2221 tgtatgcagt atgccttact taaaaagatt aaaaggatac taatgttaaa tcttctagga
2281 aatagaacct agacttcaaa gccagtattt gtttaggtca tgaagcaaac aatgctctaa
2341 tcacaatatt aactgtttaa ttaaaatgtt gtaacaagta taaaacaggg aatgtaagtt
2401 tattaccaaa gtgatatgta ttccaaaaaa gtcatagaag atgaagcact ataatattgt
2461 tcccatatat ttaaaatacc caagtacatt ctaattacca gtatatcaga ggaaaatttt
2521 cgtagtcttt gtaaaataat atactcatca tagaaaactt gaaaaatgca gaaatgtata
2581 aaaaagcaaa aatgattact gataatatca caacccagaa gtaaccacct ttaaaaagca
2641 acccccatgt atgcctatat gtgtattgta tacttttttt acataattgg agtcatactg
2701 taaacagttt tataagtaga tctttttcat tgcaaaattg ccacattttc ttatggcatt
2761 aaaaatttta caaaaacata attttaatgg ctatattata ttccatttaa tggatgcaac
2821 tcagtttatt taaccattcc catgttgtta actatttagg ttgtttctaa ttttcattat
2881 tataaagttg cagaaatttg gtgtacataa aactgtctcc atataattga ttattaggat
2941 atattcccat gaaggattct ttttttaaaa aaatgtgaaa tgtcatcttg tacttacacc
3001 tttcatgaaa agggatttcc tgcttttgta ctgcatgggt ggcagttgtg aggaaaagcc
3061 agtcaaatga cctttttaca aaagaaatgc agtggtcact tcagttgaga gtgacttttt
3121 aatacaacaa gatcaactag aagaattcaa ctgtctcaag aatcaaggta ccccaatata
3181 tctcgcaatt ccaaactttg tttgagggac tcgttatcca gctcttggta gccacacctg
3241 caatgtaaaa tggaagaaaa tgcaaagaaa ccaaatgtgc cgagtgaata aaggattgtc

3301 atatcaatta gtgtgggtcc atctgtagca aatgggttga gtgtgtcagt atgtggttgg
3361 ttactgtgta ttcgccagga atcaccccga taggctgcca ccctattagg tgatacctgt
3421 ttaatatgtt gtccaggtag actagtagtt gcatcagttt gctgtaacaa gtaaccagtg
3481 aggtaacaca gtggtgaagc aggtcagggg aggtcaggag gatgtctgag agaaagaagt
3541 ccgggagatg aatggctgtc taggaaggag gatgtcagtg cacggttagt gtttgagcag
3601 agggcagact tgtaaagtac ctgtagtgaa aagaatgtgg ggacccgatt agcagaaagg
3661 tgtttgcacg tactttatac aaaatacaga atactttata ttggaagtga aagaaatgaa
3721 cgtggacttt tacacatgtg catatttttc tggaggctat atggattatt tgaaatgaaa
3781 agcatactaa ttggacttta ataaatcatt ttcaatca

### Cynomolgus Monkey LPAR1 mRNA (SEQ ID NO. 2, NCBI Accession No. XM_005581086.3)

1 ggctgacgct ccccgagtgg ctactccgtc tgcagctccg ctgctgagcg cgggcgggat
61 ctggggcggc cagggcccag agccgcggag ccctccccgc cgcccggctg agcacgggat
121 cccggcgggg tgggcgcagg gggcggcctc gctctgggcg acggccgagg ggcgggcgga
181 gggacgggct cggctggcgg cggggcgggg gccgccggga ctgggcgcgc ggcctgaagc
241 caacccgggg gcggcgggag cgggacgcgc ggcggctgca agcgcaggta aaggggcagg
301 cgcggcgtgt ggggacggcg ccccctgggc gcgaagccag aggccgcggc gacggctcgt
361 tagccacacg gcgcgctggg ctcacactgt cccgcggtgg acgggctttg tggttggggg
421 cgcgcgtgcg ggtgccagag agagtgtgag tgcgcgcgct gggccgcggc gcgggtgggt
481 ggccgtgcgt tcttactagc cggcctgcag gaggcgaggc tccccggacc tcccgcaccc
541 agcggctgac cgatcctcgg gagggaagtt gccgcaaccg cccgggccgc cggccctcct
601 gtctcgctcc aggtggacat ctgatttatg aagctcccca tccacctgtc tgagtacctg
661 acttctcagg actgactcct acagcatcag atacacagct tctcctagca tgaccttgat
721 ctgatcagca aacaagaaca tttgtctccc gtagttctgg ggcctattca ccatctacaa
781 ccacagagct gtcatggctg ccatctctac ttccatccct gtaatttcac agccccagtt
841 cacagccatg aatgaaccac agtgcttcta caacgaatcc attgccttct tttataaccg
901 aagtggaaag catcttgcca cagaatggaa cacagtcagc aagctggtga tgggacttgg
961 aatcactgtt tgtatcttca tcatgttggc caacctactg gtcatggtgg caatctatgt
1021 caaccgccgc ttccattttc ctatttatta cctaatggct aatctggctg ctgcagactt
1081 ctttgctggg ttggcctact tctatctcat gttcaacaca gggcccaata ctcggaggct
1141 gactgttagc acatggctcc ttcgtcaggg cctcattgac accagcctga cagcatctgt
1201 ggccaactta ctggctattg caatcgagag gcacattacg gttttccgca tgcagctcca
1261 cacacggatg agcaaccggc gggtagtggt ggtcattgtg gtcatctgga ctatggccat
1321 cgttatgggt gctataccca gtgtgggctg gaactgtatc tgtgatattg aaaattgttc
1381 caacatggca cccctgtaca gcgactctta cttagtcttc tgggccattt tcaacttggt
1441 gacctttgtg gtaatggtgg ttctctatgc tcacatcttt ggctatgttc gccagaggac
1501 tatgagaatg tctcggcata gttctggacc ccggcggaat cgggatacta tgatgagtct
1561 tctgaagact gtggtcattg tgcttggggc ctttatcatc tgctggactc ctggattggt
1621 tttgttactt ctagacgtgt gttgtccgca gtgcgacgtg ctggcctatg agaaattctt
1681 ccttctcctt gctgaattca actctgccat gaaccccatc atttactcct accgcgacaa
1741 agaaatgagc gccaccttta ggcagatcct ctgctgccaa cgcagtgaga accccagcgg
1801 ccccacagaa ggctcagacc gctcggcttc ctccctcaac cacaccatct tggctggagt
1861 tcacagcaat gaccactctg tggtttagaa cggaaactga gatgaggaac cagccgtcct
1921 ctcttggagg ataaacagtc tccccctacc caattgccag ggcaaggtgg ggtgtgagag
1981 aggagaaatg tcaactcatg tacttaaaca ctaaccaatg acagtatttg ttcctaggcc
2041 ccacaagact tgatatatat tgaaaattag cttatgtgac aaccctcacc ttgatcccca
2101 tcccttctga aagtaggaag tggagctctt gcaatggaat tcaagaacag actctggagt
2161 gtccatttag actacactaa ctagacttta aaaagatttt gtgtggtttg gtgcaagtca
2221 ggataaattc tgactagttg aatccacagc ttcatttata tacagccttc cctttttat
2281 ttttaaagga tacgtttcac ttaataaaca tgtttatgcc tatcagcatg tttgtgatgg
2341 atgagactat ggactgtttt taaactacca taattccatt ttttccctta cataggaaaa
2401 ctgtaagctg gaattatctt ttgtttagaa agcatgcatg taatgtatgt atgcagtatg

2461 ccttacttaa aaagattaaa aggatactaa ttttaaatct tctaggaaat agaacctaga
2521 cttcaaagcc agtatttgtt taggttatga agcaaacaat gctctaatga caatattaac
2581 tgtttaatta aaatgttgta acaagtttaa aacagggaat gtaaattttt gccaaagtga
2641 tatgtattcc aaaaaagtca tagaagatga agcactgtaa tattgttccc acatatttaa
2701 aatacccaag tacattctaa ttaccagtgt atcagaggaa agttttcgta gtctttgtaa
2761 aataatatac tcatcataga aaacttgaaa aatgcagaaa tgtataaaaa agcaaaaatt
2821 gttactgata atatcacaac ccagaaggaa ccacctttca aaagcaaccc ccatatatgc
2881 ctatgtatgt attgtatacc ttttttacat aattggaatc atactgtaaa cagttttata
2941 agtagatctt tttcattgca aaattgccac attttcttat ggtattaaaa attttacaaa
3001 aacaatttta atggctatat tatattccat tgaatggatg caactcagtt tatttaacca
3061 ttcccatgtt gttagctgtt taggttattt ctaattttca ttttgcagaa atttgtgtg
3121 cataaacttt gtccatataa ttgattatta ggacatattc ccatgaagga ttcttttttt
3181 caaaaaatgt gaaatgtcat cttttactta tacctttcat aaaaagggat ttcctgcgtt
3241 tgcactgcat gggtggcagt tgtgaggaaa agccagtcaa atgacctttt tacaaaagaa
3301 atgcagtggt cagtttagtt gagagtgact ttttaatata acaagatcaa ctagaagcat
3361 tcaactgtct caagagtcaa ggtatcccaa tatatctcgc aattccaaac tttgtttgag
3421 tgactcatta tccagctctt ggtagccaca cctgcaatgt aaaattgaag aaaattcaaa
3481 gaaaccaaat gtgctgagtg aataaaggat tgtcatatca attagtttgg gtctatctgt
3541 agcaaatggg ttgagtgtgt cagtaggtga ttggttactg tgtattctcc aggaatcacc
3601 ccaataggct gctgccctat taggtgatac ctgtttaata cgttgtccag gtagagtagt
3661 agttgcatca gtttgctgta acaagtgacc agtgaagtaa cacagtggtg aagcaggtca
3721 ggggaggtca ggaggatgtc tgagagaaag aagtctggga gatgaatgac tgtctaggaa
3781 ggaggacatc agtgcacggt tactgtttga gcagagggca gacttgtaaa gtacccgtag
3841 tgaaaagaat gtggggactc aattagcaga aaggtgtttg cacatacttt ttacaaaata
3901 cagaatactt tatattggaa gtgaaagaaa tgaacttgga cttttacgca tgtgcatatt
3961 tttctggagg ctgtatggat tatttgaaat gaaaagcata ctaattggac tttaataaat
4021 cattttcaat cagcgcttgg taa

### Examples

### Experiment Methods and Materials

### Target Sequences Screening

The siRNAs were designed based on the full-length mRNA sequence of LPAR1 (NM_001351411.2), and all sequences were derived from the NCBI gene database. All siRNAs were designed to ensure homology with the sequences of human (Gene ID : 1902, NM_001351411.2, SEQ ID NO. 1) and cynomolgus monkey (Gene ID: 101864894, XM_005581086.3, SEQ ID NO. 2).

### Synthesis of Oligonucleotides

The synthesis method for the oligonucleotides is similar to the conventional phosphoramidite solid-phase synthesis method (all were synthesized by Suzhou Biosyntech Co., Ltd.), which includes four steps: deprotection, coupling, capping, and oxidation or sulfurization. The solid support was used as the starting cycle, and nucleoside monomers were sequentially linked in the 3' to 5' direction. The nucleoside phosphoramidite monomer raw materials, including 2'-OTBDMS, 2'-F RNA, 2'-OMe RNA, etc., were purchased from Hongene Biotech Corporation. After completion of solid-phase synthesis, the solid support was transferred to a centrifuge tube, and a 3:1 mixture of 28% ammonium hydroxide and ethanol was added to soak the support at 50 °C for 16 hours to cleave the oligonucleotides from the solid support into solution. After centrifugation, the supernatant was transferred to another centrifuge tube, concentrated to dryness, and then redissolved in deionized water. Purification was performed using a C18 reversed-phase column with 0.1 M TEAA and acetonitrile as the mobile phase. The target oligonucleotide was collected, lyophilized, identified as the desired product by LC-MS, and quantified by UV absorbance at 260 nm. The siRNA was obtained by annealing the sense strand and the antisense strand.

### Screening of in vitro inhibitory activity of unmodified siRNAs against LPAR1 mRNA in the A549 cell line.

### Dissolution of the compound

The tube containing the compound dry powder was placed in a centrifuge and centrifuged at 12,000 rpm for 5 min. An appropriate amount of RNase-free deionized water was then added, and the mixture was mixed thoroughly to prepare a 20 µM stock solution. The stock solution of each compound was aliquoted into 3 tubes (10 µL per tube) and stored at - 20 °C in a refrigerator.

### Reverse Transfection

Dilution of the compound: The 20 µM stock solution of the compound was diluted with Opti-MEM reduced-serum medium to 12 times the final working concentration and set aside.

Transfection reagent dilution: The transfection reagent was diluted with Opti-MEM reduced-serum medium at a ratio of 98.5:1.5, gently mixed, and incubated at room temperature for 15 min.

Preparation of transfection complexes: Equal volumes of the diluted compound and the diluted transfection reagent were mixed, gently vortexed, and incubated at room temperature for 15 min. Then, 20 µL of the transfection complexes were added to each well of a 96-well cell culture plate.

Cell seeding: A549 cells at approximately 80% confluency were selected, trypsinized, and resuspended in complete medium without penicillin-streptomycin. Cell counting was performed, and a cell suspension at a concentration of 100,000 cells/mL was prepared. Subsequently, 100 µL of the cell suspension was added to each well of the plate containing the transfection complexes.

The cells were cultured at 37 °C in a 5% CO₂ incubator for 24 hours.

### Cell Lysis

The cell lysis buffer was prepared according to the table below.

| Reagents | Volume (µl) |
|---|---|
| FlysisAmp cell lysis buffer | 46 |
| DNase I | 2 |
| Enhancer Solution | 2 |

The cell culture plate was taken out, the medium was discarded, and the cells were washed once with pre-chilled DPBS. Then, 50 µL of cell lysis buffer was added to each well, mixed by pipetting 5-10 times, and incubated at room temperature for 10 min. Subsequently, 5 µL of cell lysis stop solution was added to each well, mixed by pipetting 5-10 times, and incubated at room temperature for 2 min to stop the reaction. The cell plate was placed on ice for later use.

### Real-time fluorescent quantitative PCR detection

### Quantitative PCR reaction Mix

| Reagents | Volume (µl) |
|---|---|
| 5X 1-Step SYBR Green Mix (ROX) | 2 |
| F Primer (10 µM) | 0.2 |
| R Primer (10 µM) | 0.2 |
| Template (cell lysate) | 1 |
| ddH₂O (RNase free) | 6.6 |

The quantitative PCR reaction mix was prepared according to the table above, and 9 µL thereof was added to each well of a 384-well plate. Then, 1 µL of cell lysate was added as template. The PCR amplification was performed according to the cycling program shown in the table below.

| Phases | | Temperature | Duration |
|---|---|---|---|
| Pre-denaturation | | 95 °C | 30 s |
| Cycles (40 cycles) | Denaturation | 95 °C | 10 s |
| | Annealing | 60 °C | 30 s |
| Melting Curve | | 95 °C | 15 s |
| | | 60 °C | 60 s |
| | | 95 °C | 15 s |

### Data Analysis

The relative expression levels of genes were analyzed using the relative quantitative PCR method (2^{-ΔΔCt}).

To evaluate the *in vitro* inhibitory activity of the unmodified siRNA compounds against LPAR1 mRNA in the A549 cell line, the designed siRNA compounds were subjected to *in vitro* functional evaluation, and the results were presented in Table 2.

**Table 2. In vitro inhibitory activity of unmodified siRNA compounds against LPAR1 mRNA in the A549 cell line.**

| Compound ID | Average Remaining | | SD | |
|---|---|---|---|---|
| | 10 nM | 50 nM | 10 nM | 50 nM |
| 8033 | 0.17 | 0.18 | 0.03 | 0.01 |
| 8034 | 0.19 | 0.21 | 0.02 | 0.00 |
| 8035 | 0.44 | 0.61 | 0.01 | 0.04 |
| 8064 | 0.20 | 0.20 | 0.01 | 0.01 |
| 8065 | 0.15 | 0.16 | 0.01 | 0.01 |
| 8066 | 0.16 | 0.16 | 0.02 | 0.02 |
| 8067 | 0.14 | 0.15 | 0.00 | 0.01 |
| 8068 | 0.16 | 0.19 | 0.02 | 0.01 |
| 8069 | 0.19 | 0.26 | 0.02 | 0.01 |

To evaluate the *in vitro* inhibitory activity of the unmodified siRNA compounds against LPAR1 mRNA in the A549 cell line, the designed siRNA compounds were subjected to *in vitro* functional evaluation, and the results were presented in Table 3.

**Table 3. In vitro inhibitory activity of unmodified siRNA compounds against LPAR1 mRNA in the A549 cell line.**

| Compound ID | Average Remaining | | SD | |
|---|---|---|---|---|
| | 10 nM | 1 nM | 10 nM | 1 nM |
| 8033 | 0.23 | 0.30 | 0.03 | 0.05 |
| 8070 | 0.22 | 0.34 | 0.04 | 0.07 |
| 8089 | 0.18 | 0.27 | 0.04 | 0.06 |
| 8090 | 0.50 | 0.53 | 0.05 | 0.08 |
| 8091 | 0.23 | 0.29 | 0.03 | 0.02 |
| 8105 | 0.22 | 0.32 | 0.04 | 0.04 |
| 8108 | 0.26 | 0.36 | 0.03 | 0.05 |
| 8110 | 0.33 | 0.40 | 0.06 | 0.08 |
| 8111 | 0.24 | 0.34 | 0.06 | 0.05 |
| 8112 | 0.30 | 0.45 | 0.04 | 0.05 |
| 8113 | 0.35 | 0.50 | 0.07 | 0.17 |
| 8114 | 0.34 | 0.49 | 0.05 | 0.11 |
| 8116 | 0.38 | 0.47 | 0.07 | 0.04 |
| 8121 | 0.26 | 0.37 | 0.03 | 0.05 |
| 8122 | 0.25 | 0.31 | 0.04 | 0.05 |
| 8139 | 0.48 | 0.46 | 0.04 | 0.10 |
| 8140 | 0.37 | 0.48 | 0.06 | 0.06 |
| 8143 | 0.55 | 0.61 | 0.04 | 0.04 |
| 8163 | 0.41 | 0.42 | 0.06 | 0.09 |
| 8290 | 0.46 | 0.49 | 0.04 | 0.03 |
| 8425 | 0.45 | 0.52 | 0.06 | 0.08 |

To evaluate the *in vitro* inhibitory activity of the unmodified siRNA compounds against LPAR1 mRNA in the A549 cell line, the designed siRNA compounds were subjected to *in vitro* functional evaluation, and the results were presented in Table 4.

**Table 4. In vitro inhibitory activity of unmodified siRNA compounds against LPAR1 mRNA in the A549 cell line.**

| Compou nd ID | Average Remaining | | | | | SD | | | | | IC50 |
|---|---|---|---|---|---|---|---|---|---|---|---|
| | 1 nM | 0.4 nM | 0.08 nM | 0.016 nM | 0.0032 nM | 1 nM | 0.4 nM | 0.08 nM | 0.016 nM | 0.003 2nM | nM |
| 8064 | 0.30 | 0.25 | 0.35 | 0.41 | 0.68 | 0.05 | 0.02 | 0.04 | 0.05 | 0.05 | 0.004 |
| 8065 | 0.22 | 0.23 | 0.30 | 0.41 | 0.69 | 0.02 | 0.03 | 0.06 | 0.03 | 0.05 | 0.005 |
| 8066 | 0.23 | 0.23 | 0.35 | 0.60 | 0.76 | 0.04 | 0.03 | 0.02 | 0.04 | 0.06 | 0.028 |
| 8067 | 0.26 | 0.23 | 0.25 | 0.38 | 0.57 | 0.03 | 0.02 | 0.02 | 0.04 | 0.07 | 0.002 |
| 8068 | 0.23 | 0.23 | 0.26 | 0.37 | 0.57 | 0.01 | 0.02 | 0.08 | 0.02 | 0.08 | 0.006 |
| 8091 | 0.36 | 0.37 | 0.41 | 0.61 | 0.82 | 0.06 | 0.04 | 0.04 | 0.02 | 0.09 | 0.016 |

It was demonstrated that the siRNAs provided by the present invention were capable of effectively reducing LPAR1 mRNA levels in the A549 cell line

## Claims

1. An RNAi agent for inhibiting expression of lysophosphatidic acid receptor 1 (LPAR1) gene in a cell, comprising a sense strand and an antisense strand forming a duplex region, wherein the antisense strand comprises at least 15, at least 16, at least 17, at least 18, at least 19, at least 20, or at least 21 contiguous nucleotides that are at least 80%, 85%, 90%, 95%, or 100% complementary to an equal-length contiguous nucleotide sequence of a target region of mRNA encoding LPAR1, or a nucleotide sequence having no more than 3 nucleotide differences therefrom.

2. The RNAi agent according to claim 1, wherein the target region is positions 602-631, 793-825, 833-859, 1107-1143, 1167-1194, 1211-1288, 1326-1360, 1489-1523, 1777-1804, 2264-2290, or 2786-2813 of the nucleotide sequence set forth in SEQ ID NO: 1.

3. The RNAi agent according to claim 1 or 2, wherein the antisense strand comprises at least 15 contiguous nucleotides of a nucleotide sequence selected from SEQ ID NOs: 40 to 76 and nucleotide sequences having 1 to 3 nucleotide differences therefrom.

4. The RNAi agent according to any one of claims 1 to 3, wherein the duplex region is 17 to 23 base pairs in length, preferably 18 to 21 base pairs, more preferably 19 base pairs.

5. The RNAi agent according to any one of claims 1 to 4, wherein each of the sense strand and the antisense strand is 17 to 23 nucleotides in length, preferably 19 to 21 nucleotides.

6. The RNAi agent according to any one of claims 1 to 5, wherein the RNAi agent comprises one or two blunt ends, preferably one blunt end.

7. The RNAi agent according to any one of claims 1 to 6, wherein the RNAi agent comprises one or two overhangs, preferably one overhang, each overhang comprising 1 to 4 unpaired nucleotides, preferably 2 unpaired nucleotides.

8. The RNAi agent according to claim 7, wherein the overhang is located at 3'-end of the sense strand, 3'-end of the antisense strand, or simultaneously at 3'-end of the sense strand and 3'-end of the antisense strand; preferably, the overhang is located at 3'-end of the antisense strand, and more preferably, the RNAi agent has one blunt end.

9. The RNAi agent according to any one of claims 1 to 8, wherein the sense strand comprises at least 15 contiguous nucleotides of any nucleotide sequence selected from SEQ ID NOs: 3 to 39 and nucleotide sequences having 1 to 3 nucleotide differences therefrom.

10. The RNAi agent according to any one of claims 1 to 9, wherein the antisense strand has no more than 23 nucleotides and comprises a nucleotide sequence selected from SEQ ID NOs: 40 to 76; and the sense strand has no more than 21 nucleotides and comprises a nucleotide sequence from SEQ ID NOs: 3 to 39.

11. The RNAi agent according to claim 10, wherein
the sense strand comprises or consists of a sequence set forth in SEQ ID NO:39 or a the sense strand comprises or consists of a sequence set forth in SEQ ID NO:3 or a nucleotide sequence having 1, 2, or 3 nucleotide differences therefrom, and the antisense strand comprises or consists of a sequence set forth in SEQ ID NO:40 or a nucleotide sequence having 1, 2, or 3 nucleotide differences therefrom;
the sense strand comprises or consists of a sequence set forth in SEQ ID NO:4 or a nucleotide sequence having 1, 2, or 3 nucleotide differences therefrom, and the antisense strand comprises or consists of a sequence set forth in SEQ ID NO:41 or a nucleotide sequence having 1, 2, or 3 nucleotide differences therefrom;
the sense strand comprises or consists of a sequence set forth in SEQ ID NO:5 or a nucleotide sequence having 1, 2, or 3 nucleotide differences therefrom, and the antisense strand comprises or consists of a sequence set forth in SEQ ID NO:42 or a nucleotide sequence having 1, 2, or 3 nucleotide differences therefrom;
the sense strand comprises or consists of a sequence set forth in SEQ ID NO:6 or a nucleotide sequence having 1, 2, or 3 nucleotide differences therefrom, and the antisense strand comprises or consists of a sequence set forth in SEQ ID NO:43 or a nucleotide sequence having 1, 2, or 3 nucleotide differences therefrom;
the sense strand comprises or consists of a sequence set forth in SEQ ID NO:7 or a nucleotide sequence having 1, 2, or 3 nucleotide differences therefrom, and the antisense strand comprises or consists of a sequence set forth in SEQ ID NO:44 or a nucleotide sequence having 1, 2, or 3 nucleotide differences therefrom;
the sense strand comprises or consists of a sequence set forth in SEQ ID NO:8 or a nucleotide sequence having 1, 2, or 3 nucleotide differences therefrom, and the antisense strand comprises or consists of a sequence set forth in SEQ ID NO:45 or a nucleotide sequence having 1, 2, or 3 nucleotide differences therefrom;
the sense strand comprises or consists of a sequence set forth in SEQ ID NO:9 or a nucleotide sequence having 1, 2, or 3 nucleotide differences therefrom, and the antisense strand comprises or consists of a sequence set forth in SEQ ID NO:46 or a nucleotide sequence having 1, 2, or 3 nucleotide differences therefrom;
the sense strand comprises or consists of a sequence set forth in SEQ ID NO:10 or a nucleotide sequence having 1, 2, or 3 nucleotide differences therefrom, and the antisense strand comprises or consists of a sequence set forth in SEQ ID NO:47 or a nucleotide sequence having 1, 2, or 3 nucleotide differences therefrom;
the sense strand comprises or consists of a sequence set forth in SEQ ID NO:11 or a nucleotide sequence having 1, 2, or 3 nucleotide differences therefrom, and the antisense strand comprises or consists of a sequence set forth in SEQ ID NO:48 or a nucleotide sequence having 1, 2, or 3 nucleotide differences therefrom;
the sense strand comprises or consists of a sequence set forth in SEQ ID NO:12 or a nucleotide sequence having 1, 2, or 3 nucleotide differences therefrom, and the antisense strand comprises or consists of a sequence set forth in SEQ ID NO:49 or a nucleotide sequence having 1, 2, or 3 nucleotide differences therefrom;
the sense strand comprises or consists of a sequence set forth in SEQ ID NO:13 or a nucleotide sequence having 1, 2, or 3 nucleotide differences therefrom, and the antisense strand comprises or consists of a sequence set forth in SEQ ID NO:50 or a nucleotide sequence having 1, 2, or 3 nucleotide differences therefrom;
the sense strand comprises or consists of a sequence set forth in SEQ ID NO:14 or a nucleotide sequence having 1, 2, or 3 nucleotide differences therefrom, and the antisense strand comprises or consists of a sequence set forth in SEQ ID NO:51 or a nucleotide sequence having 1, 2, or 3 nucleotide differences therefrom;
the sense strand comprises or consists of a sequence set forth in SEQ ID NO:15 or a nucleotide sequence having 1, 2, or 3 nucleotide differences therefrom, and the antisense strand comprises or consists of a sequence set forth in SEQ ID NO:52 or a nucleotide sequence having 1, 2, or 3 nucleotide differences therefrom;
the sense strand comprises or consists of a sequence set forth in SEQ ID NO:16 or a nucleotide sequence having 1, 2, or 3 nucleotide differences therefrom, and the antisense strand comprises or consists of a sequence set forth in SEQ ID NO:53or a nucleotide sequence having 1, 2, or 3 nucleotide differences therefrom;
the sense strand comprises or consists of a sequence set forth in SEQ ID NO:17 or a nucleotide sequence having 1, 2, or 3 nucleotide differences therefrom, and the antisense strand comprises or consists of a sequence set forth in SEQ ID NO:54or a nucleotide sequence having 1, 2, or 3 nucleotide differences therefrom;
the sense strand comprises or consists of a sequence set forth in SEQ ID NO:18 or a nucleotide sequence having 1, 2, or 3 nucleotide differences therefrom, and the antisense strand comprises or consists of a sequence set forth in SEQ ID NO:55 or a nucleotide sequence having 1, 2, or 3 nucleotide differences therefrom;
the sense strand comprises or consists of a sequence set forth in SEQ ID NO:19 or a nucleotide sequence having 1, 2, or 3 nucleotide differences therefrom, and the antisense strand comprises or consists of a sequence set forth in SEQ ID NO:56 or a nucleotide sequence having 1, 2, or 3 nucleotide differences therefrom;
the sense strand comprises or consists of a sequence set forth in SEQ ID NO:20 or a nucleotide sequence having 1, 2, or 3 nucleotide differences therefrom, and the antisense strand comprises or consists of a sequence set forth in SEQ ID NO:57 or a nucleotide sequence having 1, 2, or 3 nucleotide differences therefrom;
the sense strand comprises or consists of a sequence set forth in SEQ ID NO:21 or a nucleotide sequence having 1, 2, or 3 nucleotide differences therefrom, and the antisense strand comprises or consists of a sequence set forth in SEQ ID NO:58 or a nucleotide sequence having 1, 2, or 3 nucleotide differences therefrom;
the sense strand comprises or consists of a sequence set forth in SEQ ID NO:22 or a nucleotide sequence having 1, 2, or 3 nucleotide differences therefrom, and the antisense strand comprises or consists of a sequence set forth in SEQ ID NO:59 or a nucleotide sequence having 1, 2, or 3 nucleotide differences therefrom;
the sense strand comprises or consists of a sequence set forth in SEQ ID NO:23 or a nucleotide sequence having 1, 2, or 3 nucleotide differences therefrom, and the antisense strand comprises or consists of a sequence set forth in SEQ ID NO:60 or a nucleotide sequence having 1, 2, or 3 nucleotide differences therefrom;
the sense strand comprises or consists of a sequence set forth in SEQ ID NO:24 or a nucleotide sequence having 1, 2, or 3 nucleotide differences therefrom, and the antisense strand comprises or consists of a sequence set forth in SEQ ID NO:61 or a nucleotide sequence having 1, 2, or 3 nucleotide differences therefrom;
the sense strand comprises or consists of a sequence set forth in SEQ ID NO:25 or a nucleotide sequence having 1, 2, or 3 nucleotide differences therefrom, and the antisense strand comprises or consists of a sequence set forth in SEQ ID NO:62 or a nucleotide sequence having 1, 2, or 3 nucleotide differences therefrom;
the sense strand comprises or consists of a sequence set forth in SEQ ID NO:26 or a nucleotide sequence having 1, 2, or 3 nucleotide differences therefrom, and the antisense strand comprises or consists of a sequence set forth in SEQ ID NO:63 or a nucleotide sequence having 1, 2, or 3 nucleotide differences therefrom;
the sense strand comprises or consists of a sequence set forth in SEQ ID NO:27 or a nucleotide sequence having 1, 2, or 3 nucleotide differences therefrom, and the antisense strand comprises or consists of a sequence set forth in SEQ ID NO:64 or a nucleotide sequence having 1, 2, or 3 nucleotide differences therefrom;
the sense strand comprises or consists of a sequence set forth in SEQ ID NO:28 or a nucleotide sequence having 1, 2, or 3 nucleotide differences therefrom, and the antisense strand comprises or consists of a sequence set forth in SEQ ID NO:65 or a nucleotide sequence having 1, 2, or 3 nucleotide differences therefrom;
the sense strand comprises or consists of a sequence set forth in SEQ ID NO:29 or a nucleotide sequence having 1, 2, or 3 nucleotide differences therefrom, and the antisense strand comprises or consists of a sequence set forth in SEQ ID NO:66 or a nucleotide sequence having 1, 2, or 3 nucleotide differences therefrom;
the sense strand comprises or consists of a sequence set forth in SEQ ID NO:30 or a nucleotide sequence having 1, 2, or 3 nucleotide differences therefrom, and the antisense strand comprises or consists of a sequence set forth in SEQ ID NO:67 or a nucleotide sequence having 1, 2, or 3 nucleotide differences therefrom;
the sense strand comprises or consists of a sequence set forth in SEQ ID NO:31 or a nucleotide sequence having 1, 2, or 3 nucleotide differences therefrom, and the antisense strand comprises or consists of a sequence set forth in SEQ ID NO:68 or a nucleotide sequence having 1, 2, or 3 nucleotide differences therefrom;
the sense strand comprises or consists of a sequence set forth in SEQ ID NO:32 or a nucleotide sequence having 1, 2, or 3 nucleotide differences therefrom, and the antisense strand comprises or consists of a sequence set forth in SEQ ID NO:69 or a nucleotide sequence having 1, 2, or 3 nucleotide differences therefrom;
the sense strand comprises or consists of a sequence set forth in SEQ ID NO:33 or a nucleotide sequence having 1, 2, or 3 nucleotide differences therefrom, and the antisense strand comprises or consists of a sequence set forth in SEQ ID NO:70 or a nucleotide sequence having 1, 2, or 3 nucleotide differences therefrom;
the sense strand comprises or consists of a sequence set forth in SEQ ID NO:34 or a nucleotide sequence having 1, 2, or 3 nucleotide differences therefrom, and the antisense strand comprises or consists of a sequence set forth in SEQ ID NO:71 or a nucleotide sequence having 1, 2, or 3 nucleotide differences therefrom;
the sense strand comprises or consists of a sequence set forth in SEQ ID NO:35 or a nucleotide sequence having 1, 2, or 3 nucleotide differences therefrom, and the antisense strand comprises or consists of a sequence set forth in SEQ ID NO:72 or a nucleotide sequence having 1, 2, or 3 nucleotide differences therefrom;
the sense strand comprises or consists of a sequence set forth in SEQ ID NO:36 or a nucleotide sequence having 1, 2, or 3 nucleotide differences therefrom, and the antisense strand comprises or consists of a sequence set forth in SEQ ID NO:73 or a nucleotide sequence having 1, 2, or 3 nucleotide differences therefrom;
the sense strand comprises or consists of a sequence set forth in SEQ ID NO:37 or a nucleotide sequence having 1, 2, or 3 nucleotide differences therefrom, and the antisense strand comprises or consists of a sequence set forth in SEQ ID NO:74 or a nucleotide sequence having 1, 2, or 3 nucleotide differences therefrom;
the sense strand comprises or consists of a sequence set forth in SEQ ID NO:38 or a nucleotide sequence having 1, 2, or 3 nucleotide differences therefrom, and the antisense strand comprises or consists of a sequence set forth in SEQ ID NO:75 or a nucleotide sequence having 1, 2, or 3 nucleotide differences therefrom; or
the sense strand comprises or consists of a sequence set forth in SEQ ID NO:39 or a nucleotide sequence having 1, 2, or 3 nucleotide differences therefrom, and the antisense strand comprises or consists of a sequence set forth in SEQ ID NO:76 or a nucleotide sequence having 1, 2, or 3 nucleotide differences therefrom.

12. The RNAi agent according to claim 11, wherein the RNAi agent comprises duplex 8033, 8034, 8035, 8064, 8065, 8066, 8067, 8068, 8069, 8070, 8088, 8089, 8090, 8091, 8105, 8106, 8108, 8110, 8111, 8112, 8113, 8114, 8115, 8116, 8120, 8121, 8122, 8139, 8140, 8141, 8142, 8143, 8163, 8164, 8290, 8425, or 8426.

13. The RNAi agent according to any one of claims 1 to 12, wherein the sense strand and/or antisense strand of the RNAi agent comprises at least one modified nucleotide; the modified nucleotide is independently selected from the group consisting of: 2'-deoxythymidine (dT) nucleotide, 2'-O-methyl-modified nucleotide (2'-OMe), 2'-fluoro-modified nucleotide (2'-F), 2'-deoxy-modified nucleotide, locked nucleic acid (LNA), unlocked nucleic acid (UNA), bridged nucleic acid (BNA), glycol nucleic acid (GNA), threose nucleic acid (TNA), conformationally restricted nucleotide, constrained ethyl nucleotide (cEt), 2'-amino-modified nucleotide, 2'-O-allyl-modified nucleotide, 2'-C-alkyl-modified nucleotide, 2'-O-methoxyethyl-modified nucleotide (2'-MOE), abasic nucleotide, inverted abasic nucleotide, inverted nucleotide, morpholino nucleotide (MOP), phosphoramidate (PN), tetrahydropyran-modified nucleotide (THP), 1,5-anhydrohexitol-modified nucleotide (HNA), cyclohexenyl-modified nucleotide, nucleotide comprising a phosphorothioate group (PS), nucleotide comprising a methylphosphonate group, nucleotide comprising a 5'-phosphate, nucleotide comprising a 5'-phosphate mimic, cationic lipid-conjugated nucleotide, nucleotide comprising a 5'-vinylphosphonate (5'-VP), and combinations thereof; preferably selected from 2'-O-methyl-modified nucleotide, 2'-fluoro-modified nucleotide, nucleotide comprising a phosphorothioate internucleotide linkage, and combinations thereof; and/or preferably, each nucleotide of the sense strand and/or the antisense strand of the RNAi agent is modified.

14. The RNAi agent according to claim 13, wherein in 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides.

15. The RNAi agent according to claim 13 or 14, wherein the antisense strand comprises at least one phosphorothioate internucleotide linkage; preferably, the phosphorothioate internucleotide linkage is present at one or more of the following locations: (i) between nucleotide positions 1 and 2 at 5'-end of the antisense strand; (ii) between nucleotide positions 2 and 3 at 5'-end of the antisense strand; (iii) between nucleotide positions 1 and 2 at 3'-end of the antisense strand; and (iv) between nucleotide positions 2 and 3 at 3'-end of the antisense strand.

16. The RNAi agent according to any one of claims 13 to 15, wherein in 5' to 3' direction, the nucleotides at positions 7 and 9 of the sense strand are 2'-fluoro-modified nucleotides, and one or two of the nucleotides at positions 5, 8, and 11 of the sense strand are 2'-fluoro-modified nucleotides, while the nucleotides at remaining positions of the sense strand are all 2'-methoxy-modified nucleotides.

17. The RNAi agent according to any one of claims 13 to 16, wherein the sense strand has at least one phosphorothioate internucleoside linkage; preferably, the phosphorothioate internucleoside linkage is present at one or more of the following positions: (i) between the 1st nucleotide and the 2nd nucleotide at the 5' end of the sense strand; (ii) between the 2nd nucleotide and the 3rd nucleotide at the 5' end of the sense strand; (iii) between the 1st nucleotide and the 2nd nucleotide at the 3' end of the sense strand; and (iv) between the 2nd nucleotide and the 3rd nucleotide at the 3' end of the sense strand.

18. The RNAi agent according to claim 13, wherein the modification is selected from one of STC, ESC, Advanced ESC, ESC+, AD1-3, AD5 and GalXC.

19. The RNAi agent according to any one of claims 13 to 18, wherein the 5'-terminal nucleotide of the antisense strand contains a phosphate or phosphate analog modification, preferably 5'-VP.

20. The RNAi agent according to any one of claims 13 to 19, wherein the RNAi agent comprises a ligand targeting hepatocytes, preferably, the ligand comprises a galactose moiety, a galactosamine moiety, or an N-acetylgalactosamine moiety, more preferably, the ligand is a trivalent or tetravalent N-acetylgalactosamine moiety, and even more preferably, the hepatocyte-targeting ligand is L96, NAG25, or NAG37; alternatively, the RNAi agent comprises a ligand targeting non-hepatocyte cells, preferably a lipophilic group, an integrin ligand, or a transferrin receptor 1 ligand; the lipophilic group is preferably selected from: lipids, vitamins, steroids, C₅-C₃₀ saturated or unsaturated fatty acids, C₅-C₃₀ alkyl groups, and polypeptides comprising at least one positively charged amino acid residue; the lipophilic group is more preferably selected from cholesterol, C₁₆ saturated or unsaturated fatty acid, C₁₆ alkyl group, C₂₂ saturated or unsaturated fatty acid, or C₂₂ alkyl group.

21. A pharmaceutical composition comprising an RNAi agent according to any one of claims 1 to 20; and a pharmaceutically acceptable carrier; preferably, the pharmaceutical composition is formulated for intravenous or subcutaneous injection.

22. Use of an RNAi agent according to any one of claims 1 to 20, or a pharmaceutical composition of claim 21 in the manufacturing of a medicament for:
(i) reducing expression level of LPAR1 in a cell;
(ii) preventing or treating a disease mediated by aberrant expression levels of LPAR1; or
(iii) prevention or treatment of a disease or condition selected from fibrotic diseases (e.g., pulmonary fibrosis, hepatic fibrosis, renal fibrosis), hypertrophic cardiomyopathy, diffuse scleroderma, adult-onset scleroderma, cancers (e.g., hepatocellular carcinoma, ovarian cancer, glioblastoma), diabetic nephropathy, atherosclerosis, rheumatoid arthritis, neuropathic pain, non-alcoholic steatohepatitis, benign prostatic hyperplasia, and demyelination.

23. Use of an agent that reduces expression of the gene encoding lysophosphatidic acid receptor 1 (LPAR1) in the manufacture of a medicament for the treatment of a disease or condition treatable by LPAR1 knockdown; preferably, the agent is an RNAi agent; preferably, the disease or condition is selected from fibrotic diseases (e.g., pulmonary fibrosis, hepatic fibrosis, renal fibrosis), hypertrophic cardiomyopathy, diffuse scleroderma, adult-onset scleroderma, cancers (e.g., hepatocellular carcinoma, ovarian cancer, glioblastoma), diabetic nephropathy, atherosclerosis, rheumatoid arthritis, neuropathic pain, non-alcoholic steatohepatitis, benign prostatic hyperplasia, and demyelination.
